(19) Europäisches Patentamt
European Patent Office
Office européen des brevets

(11) Publication number: **0 089 137**
**B1**

(12) # EUROPEAN PATENT SPECIFICATION

(45) Date of publication of patent specification: **12.11.86**

(51) Int. Cl.$^4$: **C 07 D 237/24,** A 01 N 43/58

(21) Application number: **83301057.2**

(22) Date of filing: **28.02.83**

(54) **Substituted pyridazines, their use as plant growth regulators, and plant growth regulating compositions containing them.**

(30) Priority: **04.03.82 US 354845**

(43) Date of publication of application:
**21.09.83 Bulletin 83/38**

(45) Publication of the grant of the patent:
**12.11.86 Bulletin 86/46**

(84) Designated Contracting States:
**AT BE CH DE FR GB IT LI NL SE**

(56) References cited:
**EP-A-0 023 358**
**EP-A-0 025 498**
**EP-A-0 037 133**
**EP-A-0 037 134**
**EP-A-0 049 971**
**GB-A-1 596 611**

(73) Proprietor: **Rohm and Haas Company**
**Independence Mall West**
**Philadelphia, Pennsylvania 19105 (US)**

(72) Inventor: **Patterson, Dennis Ray**
**407 Washington Avenue**
**North Wales Pennsylvania 19454 (US)**
Inventor: **Weir, William David**
**98 Gable Hill Road**
**Levittown Pennsylvania 19057 (US)**

(74) Representative: **Angell, David Whilton et al**
**Rohm and Haas Company Patent Department**
**Chesterfield House Bloomsbury Way**
**London WC1A 2TP (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 089 137 B1

Courier Press, Leamington Spa, England.

**Description**

This invention concerns novel substituted pyridazines and their use as plant growth regulators, particularly as chemical sterilants for producing hybrid seed of cereal grain plants; also concerned are processes for making the new compounds and plant growth regulating compositions containing them.

In U.K. Patent Specification No. 1,596,611 there are described 1-aryl (or substituted aryl)-1,4-dihydro-4-oxo-3-carboxy-6-alkylpyridazines and derivatives thereof. These compounds are disclosed as having utility as plant growth regulators particularly as chemical hybridizing agents for the production of hybrid cereal seed. Other somewhat similar pyridazine derivatives, having similar utility are disclosed in EP—A—0037133, EP—A—0037134, EP—A—0023358, EP—A—0025498 and EP—A—0049971.

The present invention provides novel substituted pyridazines which bear some resemblance to those of the prior art compounds noted above but which are principally characterized in the nature of the groups located at the 3- and, more especially the 5-positions. The compounds of this invention contain not only a carboxy group (or derivative thereof) in the 3-position but also an acyl group (or derivative thereof) in the 5-position. Representative compounds of the new substituted pyridazines are unexpectedly safer chemical hybridizing agents as compared with the cited prior art compounds in that they have significantly reduced adverse effects as regards hybrid seed quality or amount of plant injury at doses above those required to produce maximum male sterility thus allowing for higher yields of hybrid seed.

The pyridazine compounds concerned in this invention are those of Formula (I) below.

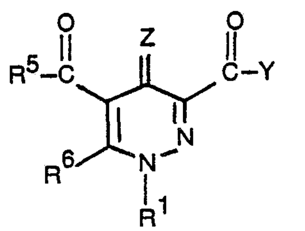

(I)

wherein:

$R^1$ is phenyl or naphthyl each optionally substituted with up to three of the same or different substituents (preferably one or two) selected from halogen (viz. Cl, Br, F and I), $(C_1—C_4)$alkyl, $(C_1—C_4)$alkoxy, ethoxycarbonyl, trihalomethyl (eg $CF_3$ or $CCl_3$), nitro and cyano;

Y is $Z^1R^3$ or $NR^7R^8$ wherein $Z^1$ is O or S; $R^3$ is hydrogen, $(C_1—C_6)$alkyl, 2-hydroxyethyl, $(C_1—C_4)$alkoxy $(C_1—C_4)$alkyl, $(C_3—C_6)$cycloalkyl, $(C_3—C_6)$cycloalkyl$(C_1—C_4)$alkyl, halo$(C_1—C_3)$alkyl containing up to three halogen atoms, phenyl or benzyl; and $R^7$ and $R^8$ are (a) the same or different and are selected from, hydrogen, $(C_1—C_4)$alkyl, $(C_1—C_4)$alkyl substituted with a carboxyl group and $(C_1—C_4)$alkoxy substituted with a carboxyl group or (b) are joined together to form an alkylene group having up to 5 carbon atoms;

Z is O or S;

$R^5$ is $(C_1—C_4)$alkyl or phenyl$(C_1—C_4)$alkyl and

$R^6$ is $(C_1—C_4)$alkyl or phenyl$(C_1—C_4)$alkyl.

Also included are agronomically acceptable derivatives of Formula I compounds which are metal or ammonium salts, acid addition salts and quaternary ammonium addition salts.

The terms "alkyl" and "alkylene" used in this specification include both straight and branched chain groups. Whenever unqualified by a prefix, a specifically named alkyl group means that group is straight chain form, for example "propyl" means "n-propyl".

Typical acid addition salts are those formed with strong acids such as HCl, HBr, $H_2SO_4$ and $HNO_3$. Typical metals salts of compounds wherein Y is OH are those of the alkali metals, particularly sodium and potassium, and less preferably the alkali metals such as calcium and magnesium.

In a preferred embodiment, the invention comprises a compound having formula I according to the invention wherein: $R^1$ is unsubstituted phenyl or phenyl substituted with up to three substituents selected from halogen, $(C_1—C_4)$alkyl, $(C_1—C_4)$alkoxy, trihalomethyl, nitro and cyano; Y is $Z^1R^3$ or $NR^7R^8$ wherein $Z^1$ is O or S, $R^3$ is hydrogen, $(C_1—C_6)$alkyl, $(C_1—C_4)$alkoxy$(C_1—C_4)$alkyl, $(C_3—C_6)$cycloalkyl, $(C_3—C_6)$cycloalkyl$(C_1—C_4)$alkyl, halo$(C_1—C_3)$alkyl containing up to three halogens, phenyl or benzyl; and $R^7$ and $R^8$ are, independently, hydrogen, $(C_1—C_4)$alkyl, $(C_1—C_4)$alkyl substituted with a carbonyl group, or $(C_1—C_4)$alkoxy substituted with a carboxyl group or, together, a $C_1—C_5$alkylene group; Z is O or S; $R^5$ is $(C_1—C_4)$alkyl or phenyl$(C_1—C_4)$alkyl; and $R^6$ is $(C_1—C_4)$alkyl or phenyl$(C_1—C_4)$alkyl; and the agronomically acceptable metal and ammonium salts, acid addition salts, and quaternary ammonium addition salts thereof.

More preferred embodiments include those compounds wherein $R^1$ is phenyl or phenyl substituted with one to three substituents selected from halogen, $(C_1—C_4)$alkyl, $(C_1—C_4)$alkoxy, trihalomethyl, nitro or cyano; Y is $Z^1R^3$ or $NR^7R^8$ wherein $Z^1$ is O, $R^3$ is $(C_1—C_4)$alkyl, alkali metal cation, alkaline earth metal cation, or ammonium or alkyl-substituted ammonium, and $R^7$ and $R^8$ are independently $(C_1—C_4)$alkyl, or $R^7$ is hydrogen and $R^8$ is $—CH_2C(O)OR^9$ wherein $R^9$ is alkali metal cation or methyl; $R^5$ is $(C_1—C_4)$alkyl; and $R^6$ is $(C_1—C_4)$alkyl.

Even more preferred compounds are those wherein $R^1$ is phenyl optionally substituted with one substituent which is Cl, Br, I, F, CN, $CF_3$, methoxy or ethoxy or two substituents which are both Cl or are one of each of Cl and $CF_3$.

Most preferred embodiments include those compounds wherein: $R^1$ is phenyl, 4-chlorophenyl, 4-bromophenyl, 4-fluorophenyl, 3,4-dichlorophenyl, 4-trifluoromethylphenyl, 4-iodophenyl or 4-methoxyphenyl; $Z^1R^3$ wherein $Z^1$ is O and $R^3$ is ($C_1$—$C_4$)alkyl ($Z^1R^3$ preferably being $OCH_3$, ONa or OK) and $R^5$ and $R^6$ are selected from ($C_2$—$C_4$)alkyl (preferably $C_2H_5$ and $C_3H_7$).

Typical compounds encompassed by this invention include:

6-ethyl-1-(4-chlorophenyl)-5-propionyl-4-oxopyridazine-3-carboxylic acid;
sodium 6-ethyl-1-(4-chlorophenyl)-5-propionyl-4-oxopyridazine-3-carboxylate;
methyl 6-ethyl-1-(4-chlorophenyl)-5-propionyl-4-oxopyridazine-3-carboxylate;
6-ethyl-1-phenyl-5-propionyl-4-oxopyridazine-3-carboxylic acid;
sodium 6-ethyl-1-phenyl-5-propionyl-4-oxopyridazine-3-carboxylate;
methyl 6-ethyl-1-phenyl-5-propionyl-4-oxopyridazine-3-carboxylate;
6-ethyl-1-(4-bromophenyl)-5-propionyl-4-oxopyridazine-3-carboxylic acid;
sodium 6-ethyl-1-(4-bromophenyl)-5-propionyl-4-oxopyridazine-3-carboxylate;
ethyl 6-ethyl-1-(4-bromophenyl)-5-propionyl-4-oxopyridazine-3-carboxylate;
6-propyl-1-(4-chlorophenyl)-5-butyryl-4-oxopyridazine-3-carboxylic acid;
sodium 6-propyl-1-(4-chlorophenyl)-5-butyryl-4-oxopyridazine-3-carboxylate;
methyl 6-propyl-1-(4-chlorophenyl)-5-butyryl-4-oxopyridazine-3-carboxylate;
potassium 6-ethyl-1-(3,4-dichlorophenyl)-5-propionyl-4-oxopyridazine-3-carboxylate;
methyl 6-ethyl-1-(3,4-dichlorophenyl)-5-propionyl-4-oxopyridazine-3-carboxylate;
potassium 6-benzyl-1-(4-methylphenyl)-5-butyryl-4-oxopyridazine-3-carboxylate;
methyl 6-benzyl-1-(4-methylphenyl)-3-butyryl-4-oxopyridazine-3-carboxylate;
potassium 6-benzyl-1-(4-trifluoromethylphenyl)-5-phenylmethylcarbonyl-4-oxopyridazine-3-carboxylate;
methyl 6-benzyl-1-(4-trifluoromethylphenyl)-5-phenyl-methylcarbonyl-4-oxopyridazine-3-carboxylate;
sodium 6-propyl-1-(4-ethoxyphenyl)-5-butyryl-4-oxopyridazine-3-carboxylate;
ethyl 6-propyl-1-(4-ethoxyphenyl)-5-butyryl-4-oxopyridazine-3-carboxylate;
6-ethyl-1-(4-chlorophenyl)-5-propionyl-4-oxopyridazine-3-(N',N'-dimethyl)carboxamide;
6-ethyl-1-phenyl-5-propionyl-4-oxopyridazine-3-(N'-carbomethoxymethyl)carboxamide;

and the various salts of the above listed compounds.

The compounds according to the invention can be obtained by the reaction sequence illustrated by the following scheme:

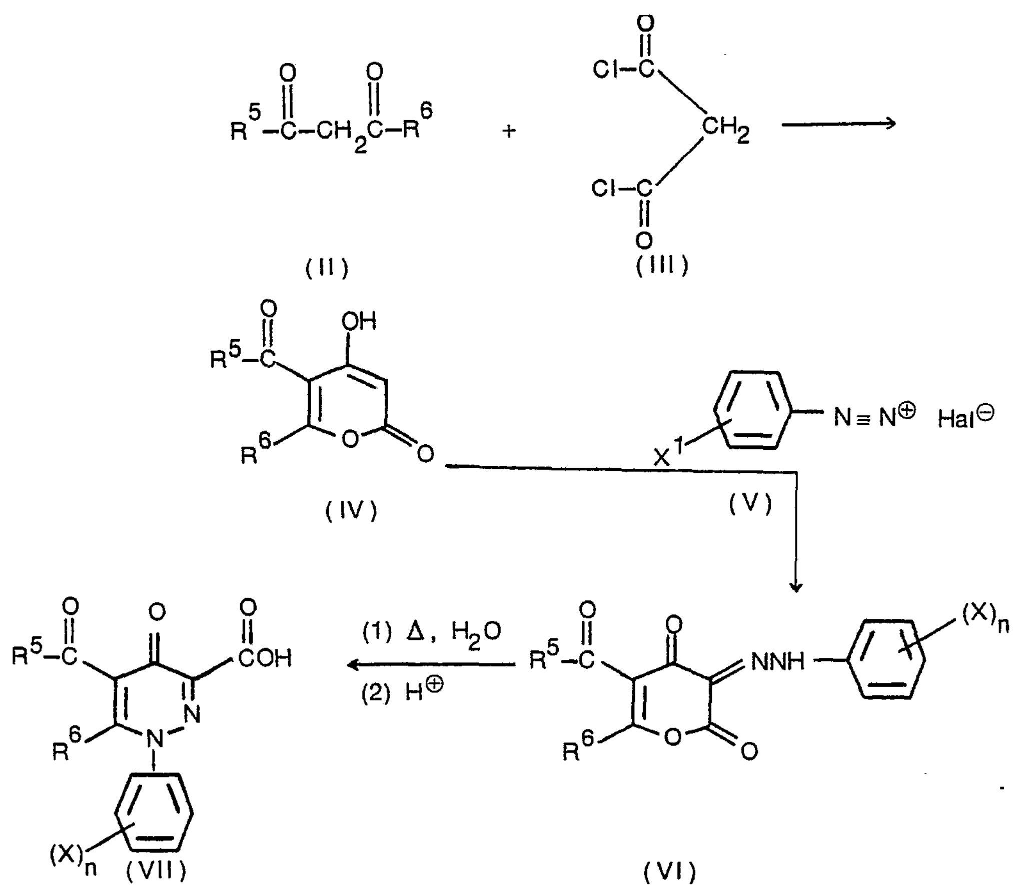


(II) (III) (IV) (V) (VI) (VII)

In the above formulae, $R^5$, $R^6$ and X are as defined for Formula I compounds, n is O or an integer of 1 to 3 and Hal is a halogen atom particularly chlorine.

Salts of the pyridazinones of formula VII can be prepared by conventional techniques, such as by neutralization with an appropriate inorganic or organic base in aqueous or alcoholic medium. For example, alkali metal salts can be prepared by treating a methanolic suspension of an appropriate 5-acyl-1-aryl-1,4-dihydro-4-oxypyridazine-3-carboxylic acid with an equivalent amount of a 50% by weight aqueous solution of the alkali hydroxide (such as sodium or potassium hydroxide), concentrating the reaction mixture, preparing a slurry of the concentrate in an inert solvent such as diethyl ether, isolating the product on a filter pad, and drying the filter cake.

Esters of pyridazinones of formula VII can be prepared by conventional techniques such as by inorganic acid catalyzed treatment of the pyridazinone with a suitable alcohol, generally in the presence of an inert co-solvent such as methylene chloride, diethyl ether, xylene and the like or by the reaction of the pyridazinone acylhalide with an appropriate alcohol, generally in the presence of an inert co-solvent, at temperatures ranging from 0—150°C. depending upon the reactants selected.

The pyridazinone carboxamide derivatives can be prepared by conventional techniques such as by reacting the pyridazinone acyl halide with an appropriate amine, monoalkylamine, dialkylamine, or carboalkoxyalkylamine in an inert solvent at temperatures of from about 0—150°C.

Acid addition salts or quaternary ammonium addition salts having the formula:

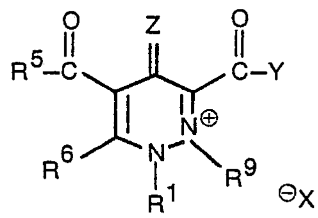

wherein $R^9$ is hydrogen or lower alkyl and X is an inorganic or organic anion such as halide, sulfate $^{1/2}$ and *p*-toluenesulfonate can be prepared by conventional techniques using for example, hydrochloric acid or methyl bromide.

The following experimental procedures are provided to illustrate processes for the preparation of compounds concerned in this invention. Parts are by weight unless otherwise specified.

Intermediate

Preparation of 6-Ethyl-4-Hydroxy-5-Propionyl Pyr-2-one

28.2 g (0.2 mol) of malonyl dichloride in 50 ml of diethyl ether is added dropwise to an ice cooled solution (0°C) of 25.6 g (0.2 mol) of 3,5-heptanedione in 100 ml of diethyl ether. The solution is stirred at 5°C for one hour and at room temperature for 18 hours. The suspension that forms is refluxed and stirred for one hour and is vacuum filtered. The filter cake is dried to afford 6-ethyl-5-hydroxy-5-propionyl pyr-2-one. A second crop is obtained by concentration of the filtrate to a volume of 50 ml. The concentrate is vacuum filtered and the filter cake dried to afford a combined yield of 11 g (28.3%), mp 133—134°C.

Example 1

Preparation of 6-Ethyl-1-(4-Chlorophenyl)-5-Propionyl-4-Oxo-Pyridazine-3-Carboxylic Acid

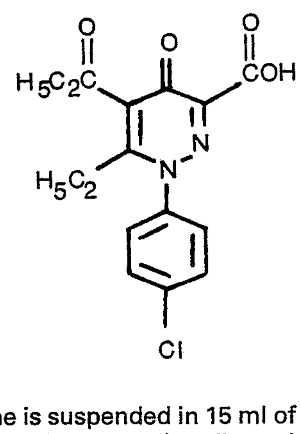

4.97 g (0.039 mol) of 4-chloroaniline is suspended in 15 ml of 12N hydrochloric acid and 30 ml of water and is cooled to 0°C. A solution of 2.97 g (0.043 mol) sodium nitrite in 10 ml of water is added dropwise while maintaining the pot temperature at 0°C to 5°C by external cooling. The diazotized *p*-chloraniline solution is added to an ice cooled solution of 7.64 g (0.039 mol) 6-ethyl-4-hydroxy-5-propionyl pyr-2-one and 17 g of sodium carbonate in 150 ml of water. The suspension that forms is stirred at 5°C for one hour,

room temperature for one hour and refluxed and stirred for two hours. The mixture is cooled and extracted with three (200 ml) portions of methylene dichloride. The aqueous solution is acidified with 12N hydrochloric acid and the suspension that forms is stirred at room temperature for 18 hours. The suspension is vacuum filtered and the filter cake is dried to afford 6 g (46% yield) of 6-ethyl-1-(4-chlorophenyl)-5-propionyl-4-oxopyridazine-3-carboxylic acid, mp 174—175°C decomposition.

Elemental analysis for $C_{16}H_{15}ClN_2O_4$:

Calc.: C, 57.40; H, 4.52; N, 8.37
Found: C, 57.49; H, 4.62; N, 8.64

Example 1a

Preparation of Sodium 6-Ethyl-1-(4-Chlorophenyl)-5-Propionyl-4-oxo-Pyridazine-3-Carboxylate

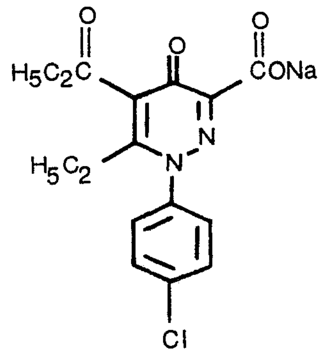

0.72 g (0.009 mol) of 50% aqueous sodium hydroxide is added to a suspension of 3.01 g (0.009 mol) 6-ethyl-1-(4-chlorophenyl)-5-propionyl-4-oxo-pyridazine-3-carboxylic acid in 25 ml of methanol. The solution that forms is concentrated *in vacuo* and the solid concentration is slurried in 50 ml of diethylether. The slurry is vacuum filtered and the filter cake is dried to afford 3 g (93.4% yield) of sodium 6-ethyl-1-(4-chlorophenyl)-5-propionyl-4-oxo-pyridazine-3-carboxylate.

Example 1b

Preparation of Potassium 6-ethyl-1-(4-Chlorophenyl)-5-Propionyl-4-Oxo-Pyridazine-3-Carboxylate

The potassium salt of 6-ethyl-1-(4-chlorophenyl)-5-propionyl-4-oxo-pyridazine-3-carboxylic acid is prepared by the procedure in Example 1a above except that an equivalent amount of aqueous potassium hydroxide is substituted for the 50% aqueous sodium hydroxide.

Example 2

Preparation of Methyl 6-Ethyl-1-(4-Chlorophenyl)-5-Propionyl-4-Oxo-Pyridazine-3-Carboxylate

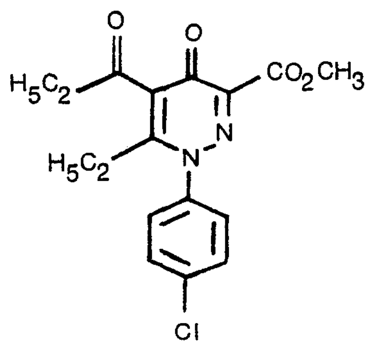

2.99 g (0.025 mol) of thionyl chloride is added dropwise to 50 ml of anhydrous methanol cooled to −5°C. The solution formed is stirred at −5°C for 5 minutes and to it there is added 2.8 g (0.0084 mol) of 6-ethyl-1-(4-chlorophenyl)-5-propionyl-4-oxo-pyridazine-3-carboxylic acid. The suspension is stirred at −5°C for one hour and at room temperature for 18 hours. The solution formed is poured into 300 ml of water and the resulting suspension is stirred at room temperature for 18 hours. The suspension is vacuum filtered and the filter cake is washed with water and dried to afford 2 g (68.3% yield) of methyl 6-ethyl-1-(4-chlorophenyl)-5-propionyl-4-oxo-pyridazine-3-carboxylate, a tan powder, mp 126—129°C decomp.

Elemental analysis for $C_{17}H_{17}ClN_2O_4$:

Calc.: C, 58.54; H, 4.91; N, 8.03
Found: C, 58.59; H, 5.03; N, 7.76

Example 3

Preparation of 6-Ethyl-1-Phenyl-5-Propionyl-4-Oxo-Pyridazine-3-Carboxylic Acid

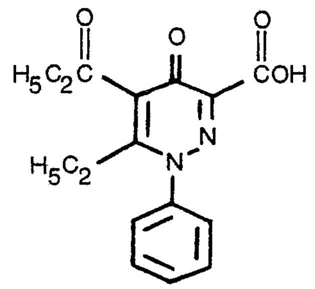

3.63 g (0.39 mol) of aniline is dissolved in 15 ml of 12N hydrochloric acid and 30 ml of water and is cooled to 0°C. A solution of 2.97 g (0.043 mol) sodium nitrite in 10 ml of water is added dropwise while maintaining the pot temperature at 0°C to 5°C by external cooling. The diazotized aniline solution is added to an ice cooled solution of 7.64 g (0.039 mol) 6-ethyl-4-hydroxy-5-propionyl pyr-2-one and 17 g of sodium carbonate in 150 ml of water. The suspension that forms is stirred at room temperature for one-half hour and is refluxed and stirred for two hours. The mixture is let stand at room temperature for 18 hours and is extracted with three (200 ml) portions of methylene dichloride. The aqueous solution is acidified to pH 1 with 12N hydrochloric acid and the suspension formed is vacuum filtered. The filter cake is washed with water and dried to afford 7.7 g (66% yield) of 6-ethyl-1-phenyl-5-propionyl-4-oxo-pyridazine-3-carboxylic acid, mp 212—213°C decomp.

Elemental analysis for $C_{16}H_{16}N_2O_4$:

| | | | |
|---|---|---|---|
| Calc.: | C, 63.99; | H, 5.37; | N, 9.33 |
| Found: | C, 63.15; | H, 5.34; | N, 8.85 |

Example 3a

Preparation of Sodium 6-Ethyl-1-Phenyl-5-Propionyl-4-Oxo-Pyridazine-3-Carboxylate

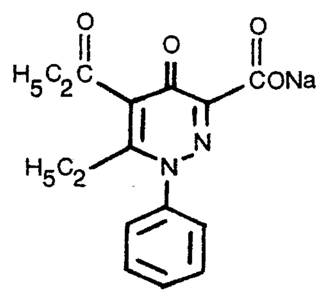

2.05 g (0.0256 mol) of 50% aqueous sodium hydroxide is added to a suspension of 7.7 g (0.0256 mol) 6-ethyl-1-phenyl-5-propionyl-4-oxo-pyridazine-3-carboxylic acid in 50 ml of anhydrous methanol. The solution formed (pH 8) is concentrated *in vacuo* and the concentrate dried to afford 7.55 g (92% yield) of sodium 6-ethyl-1-phenyl-5-propionyl-4-oxo-pyridazine-3-carboxylate, a brown powder.

Example 4

Preparation of 6-Ethyl-1-(4-Bromophenyl)-5-Propionyl-4-Oxo-Pyridazine-3-Carboxylic Acid

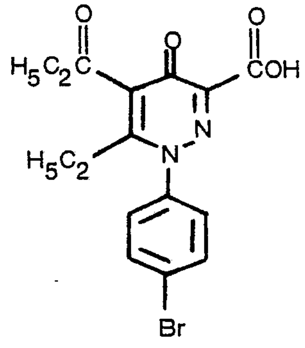

7.46 g (0.0434 mol) of *p*-bromoaniline is suspended in 17 ml of 12N hydrochloric acid and 35 ml of water and is cooled to 0°C. A solution of 3.3 g (0.048 mol) sodium nitrite in 10 ml of water is added dropwise

while maintaining the pot temperature at 0° to 5°C by external cooling. The diazotized *p*-bromoaniline solution is added to an ice cooled solution of 8.5 g (0.0434 mol) of 6-ethyl-4-hydroxy-5-propionyl pyr-2-one and 19 g of sodium carbonate in 150 ml of water. The suspension formed is stirred at room temperature for ½ hour and is refluxed and stirred for 2 hours. The mixture is extracted with three (100 ml) portions of methylene dichloride and the aqueous layer is acidified to pH 1 with 12N hydrochloric acid. The suspension formed is allowed to stand at room temperature for 18 hours and is vacuum filtered. The filter cake is washed with water and dried to afford 5.2 g (32% yield) of 6-ethyl-1-(4-bromophenyl)-5-propionyl-4-oxo-pyridazine-3-carboxylic acid, mp 184—186°C decomp.

Elemental analysis for $C_{16}H_{15}BrN_2O_4$:

Calc.: C, 50.67; H, 3.99; N, 7.39
Found: C, 50.95; H, 4.02; N, 7.30

Example 4a

Preparation of Sodium 6-Ethyl-1-(4-Bromophenyl)-5-Propionyl-4-Oxo-Pyridazine-3-Carboxylate

1.1 g (0.0137 mol) of 50% aqueous sodium hydroxide is added to a suspension of 5.2 g (0.0137 mol) of 6-ethyl-1-(4-bromophenyl)-5-propionyl-4-oxo-pyridazine-3-carboxylic acid in 50 ml of methanol. The solution formed (pH 8) is concentrated *in vacuo* and the concentrate dried to afford 5 g (91% yield) of sodium 6-ethyl-1-(4-bromophenyl)-5-propionyl-4-oxo-pyridazine-3-carboxylate, a brown powder.

Intermediate B

Preparation of 5-Butyryl-4-hydroxy-6-propyl Pyr-2-one

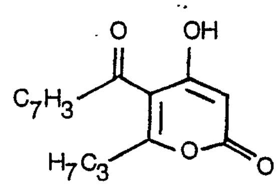

29 g (0.2 mol) of 97% malonyl dichloride dissolved in 50 ml of diethyl ether is added dropwise to an ice cooled solution (−5°C) of 31.25 g (0.2 mol) of 4,6-nonanedione in 100 ml of diethyl ether. The solution formed is stirred at 0°C. for 1 hour and at room temperature for 18 hours. The resulting suspension is refluxed and stirred for 1 hour, cooled to room temperature and vacuum filtered. The filter cake is washed with diethyl ether and dried to afford 16.3 g (36.3% yield) of 5-butyryl-4-hydroxy-5-propyl-pyr-2-one, mp 120—124°C.

Example 5

Preparation of 5-Butyryl-1-(4-Chlorophenyl)-6-Propyl-4-Oxopyridazine-3-Carboxylic Acid.

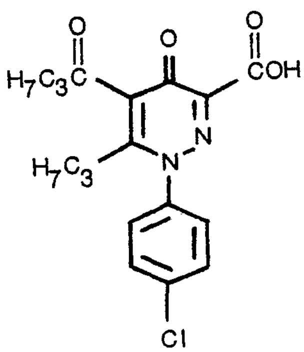

4.97 g (0.039 mol) of 4-chloroaniline is suspended in 15 ml of 12N hydrochloric acid and 30 ml of water and is cooled to 0°C. A solution of 2.97 g (0.043 mol) of sodium nitrite in 10 ml of water is added dropwise while maintaining the pot temperature at 0°C to 5°C by external cooling. The diazotized 4-chloroaniline solution is added to an ice cooled mixture of 8.75 g (0.039 mol) 5-butyryl-4-hydroxy-6-propyl pyr-2-one and 17 g of sodium carbonate in 150 ml of water and 20 ml of ethanol. The suspension formed is stirred at 0°C for one hour and refluxed and stirred for 3 hours. This mixture is allowed to stand at room temperature for 18 hours and is extracted with three portions (100 ml) of methylene dichloride. The aqueous solution is acidified to pH 1 with 12N hydrochloric acid and the suspension formed is allowed to stand at room temperature for 18 hours. The suspension is vacuum filtered and the filter cake is dried to afford 1.5 g (10.6% yield) of 5-butyryl-1-(4-chlorophenyl)-6-propyl-4-oxo-pyridazine-3-carboxylic acid, mp 178—182°C decomp.

Elemental analaysis for $C_{18}H_{19}ClN_2O_4$:

Calc.: C, 59.58; H, 5.28; N, 7.72
Found: C, 59.68; H, 5.42; N 7.42

Example 5a

Preparation of Sodium 5-Butyryl-1-(4-Chlorophenyl)-6-Propyl-4-Oxo-Pyridazine-3-Carboxylate

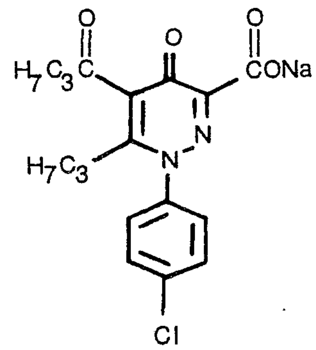

0.29 g (0.0036 mol) of 50% aqueous sodium hydroxide is added to a suspension of 1.3 g (0.0036 mol) of 5-butyryl-1-(4-chlorophenyl)-6-propyl-4-oxo-pyridazine-3-carboxylic acid in 25 ml of methanol. The solution formed (pH 8) is concentrated *in vacuo* and the concentrate dried to afford 1.3 g (94% yield) of sodium 5-butyryl-1-(4-chlorophenyl)-6-propyl-4-oxo-pyridazine-3-carboxylate, a brown powder.

The compounds of the invention are particularly useful as chemical hybridizing agents in gramineous crops such as wheat, barley, maize, rice, sorghum, millet, oats, rye, triticale, forage crops, and the like. When used as chemical hybridization agents, the compounds effectively induce a high dgree of selective male sterility without also inducing significant female sterility in the treated plants and without causing significant growth inhibition of the treated plants. As used herein, the term male sterility includes both actual male sterility as evidenced by a lack of male flower parts or by sterile pollen, and functional male sterility in which the male flower parts are unable to cause pollination. The compounds of the invention also cause other plant growth regulatory responses such as, for example, control of flowering, control of fruiting and inhibition of seed formation in non-cereal species, and other related growth regulatory responses as well as herbicidal effects.

The compounds concerned in this invention are most noteworthy in their utility in a method for the production of hybrid seed. Such method comprises treating a male/female parent of the gramineous plant prior to meiosis with the growth regulating compound, causing the plant in which male sterility has been induced to be pollinated with pollen from a male parent of a different gramineous plant and which is such as to yield the desired hybrid seeds, allowing the pollinated plant to mature until seed formation and collecting the mature hybrid seeds.

Where, using the compounds provided by the invention, male sterility is accompanied by some female infertility, the compounds are still of utility in producing new plant hybrids or in the production of ergot for which see French Published Patent Application No. 2400832.

The compounds of Formula I and their various salts are generally employed in plant growth regulating compositions in an amount from 0.1 to 95% by weight, preferably 1 to 95% by weight, the balance of the compositions comprising or consisting of an agronomically acceptable diluent or carrier. The content, in such compositions, of compound(s) of Formula I, or salts, may for example be 5%, 25% or 50% by weight. By "agronomically acceptable diluent or carrier" is meant any substance which can be used to dissolve, disperse or diffuse the active ingredient(s) without impairing the effectiveness of the active ingredient(s) and which by itself or combination with the active ingredient(s) has no significant effect on the soil, application equipment, crops or the agronomic environment.

When used as plant growth regulators, the compounds of the invention are applied in any amount which will be sufficient to effect the desired plant response. For example, when the compounds of the invention are used as chemical hybridization agents, they are generally applied to the crops to be treated at doses of about 0.03 to about 22 kg/ha (about $\frac{1}{32}$ to about 20 pounds per acre), preferably about 0.14 to about 11 kg/ha (about $\frac{1}{8}$ to about 10 pounds per acre). The rate of application will vary depending on the crop being treated, the compound being used for treatment, and related factors.

To obtain a hybrid seed, the following procedure is generally employed. The two parents to be crossed are planted in alternative strips. The male/female parent is treated with a compound of the invention. The male-sterile female parent thus produced will be pollinated by pollen from the other male-fertile male parent, and the seed produced by the female parent will be hybrid seed which can be harvested by conventional means.

A preferred method of applying a compound of the invention as a chemical hybridization agent is by foliar application. When this method is employed, selective male sterility is most effectively induced when the compound is applied between flower initiation and meiosis. The compounds of the invention may also be applied as a seed treatment by soaking the seed in a liquid formulation containing the active compound or by coating the seed with the compound. In seed treatment applications, the compounds of the invention

will generally be applied at doses of about 0.11 to about 4.5 kg per 45.4 kg of seed. The compounds of the invention are also effective when applied to the soil or to the water surface in rice crops.

The compounds of the invention can be used as plant growth regulators either individually or in mixtures. For example they can be used in combination with other plant growth regulators, such as auxins, gibberellins, ethylene-releasing agents such as ethephon, pyridones, cytokinins, maleic hydrazide, succinic acid, 2,2-dimethylhydrazide, choline and its salts, (2-chloro-ethyl) trimethylammonium chloride, triiodobenzoic acid, tributyl-2,4-dichlorobenzylphosphonium chloride, polymeric N-vinyl-2-oxazolidinones, tri(dimethylaminoethyl) phosphate and its salts, and N-dimethylamino-1,2,3,6-tetrahydrophthalamic acid and its salts, and the like, and under some conditions they may be used advantageously with other agricultural chemicals such as herbicides, fungicides, insecticides, and plant bactericides.

The compositions of the invention can be either solid or liquid formulations or solutions. For example, the compounds can be formulated as wettable powders, emulsifiable concentrates, dusts, granular formulations, aerosols, or flowable emulsion concentrates. In such formulations, the compounds are extended with a liquid or solid carrier and, when desired suitable surfactants are incorporated.

It is usually desirable, particularly in foliar applications, to include adjuvants, such as wetting agents, spreading agents, dispersing agents, stickers, adhesives, and the like, in accordance with agricultural practices. Examples of adjuvants which are commonly used in the art can be found in the John W. McCutcheon, Inc. publication "Detergents and Emulsifiers Annual."

The compounds of the invention can be dissolved in any appropriate solvent. Examples of solvents which are depending on the solubility of the active ingredient(s) useful in the practice of this invention include water, alcohols, ketones, dimethylformamide, dioxane, dimethyl sulfoxide, and the like. Mixtures of these solvents can also be used. The concentration of active ingredient(s) the solution can vary from about 2% to about 98% by weight with a preferred range being about 20% to about 75%.

For the preparation of emulsifiable concentrates, the compound can be dissolved in a solvent together with an emulsifying agent or surfactant which permits dispersion in water. Suitable emulsifiers include, for example, the ethylene oxide derivatives of alkylphenols or long-chain alcohols, mercaptans, carboxylic acids, and reactive amines and partially esterified polyhydric alcohols. Solvent-soluble sulfates or sulfonates, such as the alkaline earth metal salts or amine salts of alkylbenzenesulfonates and the fatty alcohol sodium sulfates, having surface-active properties can be used as emulsifiers either alone or in conjunction with an ethylene oxide reaction product. Flowable emulsion concentrates are formulated similarly to the emulsifiable concentrates and include, in addition to the above components, water and a stabilizing agent such as a water-soluble cellulose derivative or a water-soluble salt of a polyacrylic acid. The concentration of the active ingredient in emulsifiable concentrates is usually about 10% to 60% by weight and, in flowable emulsion concentrates, this can be as high as about 75%.

Wettable powders suitable for spraying can be prepared by admixing the compound with a finely divided solid, such as clays, inorganic silicates and carbonates, and silicas, and incorporating wetting agents, sticking agents, and/or dispersing agents in such mixtures. The concentration of active ingredients in such formulations is usually in the range of about 20% to 98% by weight, preferably about 40% to 75%. A dispersing agent may generally constitute about 0.5% to about 3% by weight of the composition, and a wetting agent may generally constitute from about 0.1% to about 5% by weight of the composition.

Dusts can be prepared by mixing the compounds of the invention with finely divided inert solids which may be organic or inorganic in nature. Materials used for this purpose include, for example, botanical flours, silicas, silicates, carbonates and clays. One convenient method of preparing a dust is to dilute a wettable powder with a finely divided carrier. Dust concentrates containing about 20% to 80% of the active ingredient are commonly made and are subsequently diluted to about 1% to 10% by weight use concentrations.

Granular formulations can be prepared by impregnating a solid such as granular Fuller's earth, vermiculite, ground corn cobs, seed hulls, including bran or other grain hulls, or similar material. A solution of one or more of the compounds in a volatile organic solvent can be sprayed or mixed with the granular solid and the solvent then removed by evaporation. The active compound will usually comprise about 2 to 15% by weight of the granular formulation.

Salts of the compounds of the invention can be formulated and applied as aqueous solutions. The salt will typically comprise about 0.1 to about 50% by weight, preferably about 0.1% to about 10%, of the solution. These compositions can also be further diluted with water if desired prior to actual application. In some applications, the activity of these compositions can be enhanced by incorporating into the composition an adjuvant such as glycerin, methylethylcellulose, hydroxyethylcellulose, polyoxyethylenesorbitan monooleate, polypropylene glycol, polyacrylic acid, polyethylene sodium malate, polyethylene oxide, or the like. The adjuvant will generally comprise about 0.1 to about 5% by weight, preferably about 0.5 to about 2%, of the composition. Such compositions can also, optionally, include an agronomically-acceptable surfactant.

The compounds of the invention can be applied as sprays by methods commonly employed, such as conventional hydraulic sprays, aerial sprays, and dusts. For low-volume applications a solution of the compound is usuaally used. The dilution and volume of application will usually depend upon such factors as the type of equipment employed, the method of application, the area to be treated and the type and stage of development of the crop being treated.

Table I below gives the structures of some representative compounds provided by the invention the preparation of some of which are set forth in the above preparative Examples. Tables II and III set forth the analytical and nuclear magnetic resonance (nmr) data of the compounds listed in Table I.

TABLE I

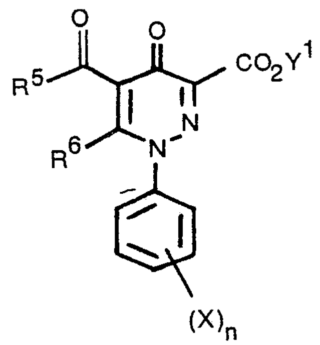

| Example No. | $Y^1$ | $R^5$ | $R^6$ | $(X)_n$ | m.p. (°C)* |
|---|---|---|---|---|---|
| 1a(1b) | Na(K) | Ethyl | Ethyl | 4-Cl | 174—75 (dec) |
| 2 | $CH_3$ | Ethyl | Ethyl | 4-Cl | 126—29 (dec) |
| 3a | Na | Ethyl | Ethyl | H | 212—13 (dec) |
| 3b | K | Ethyl | Ethyl | H | 212—13 (dec) |
| 4a | Na | Ethyl | Ethyl | 4-Br | 184—86 (dec) |
| 4b | K | Ethyl | Ethyl | 4-Br | 184—86 (dec) |
| 5a | Na | n-Propyl | n-Propyl | 4-Cl | 178—82 (dec) |
| 5b | K | n-Propyl | n-Propyl | 4-Cl | 178—82 (dec) |
| 6a | K | Ethyl | Ethyl | 3-Br | 169—70 |
| 6b | Na | Ethyl | Ethyl | 3-Br | 169-70 |
| 7a | K | Ethyl | Ethyl | 2,4-diCl | 216—17 |
| 7b | Na | Ethyl | Ethyl | 2,4-diCl | 216—17 |
| 8 | K | Ethyl | Ethyl | 3,4-di$CH_3$ | 173—74 |
| 9a | K | Ethyl | Ethyl | 4-$OCH_3$ | 133—34 |
| 9b | Na | Ethyl | Ethyl | 4-$OCH_3$ | 133—34 |
| 10 | K | Ethyl | Ethyl | 4-$CO_2$Et | 147—48 |
| 11 | K | Ethyl | Ethyl | 4-$CH_3$ | 188—89 |
| 12a | K | Ethyl | Ethyl | 4-$CF_3$ | 170—71 |
| 12b | Na | Ethyl | Ethyl | 4-$CF_3$ | 170—71 |
| 13a | K | Ethyl | Ethyl | 4-F | 180—82 |
| 13b | Na | Ethyl | Ethyl | 4-F | 180—82 |
| 14 | K | Ethyl | Ethyl | 4-CN | 200—02 |
| 15 | K | Ethyl | Ethyl | 4-OEt | 142—43 |

TABLE I (Continued)

| Example No. | $Y^1$ | $R^5$ | $R^6$ | $(X)_n$ | m.p.(°C)* |
|---|---|---|---|---|---|
| 16 | K | Ethyl | Ethyl | 4-$NO_2$ | 204—06 |
| 17a | K | Ethyl | Ethyl | 3-$CF_3$ | 110—15 |
| 17b | Na | Ethyl | Ethyl | 3-$CF_3$ | 110—15 |
| 18a | K | Ethyl | Ethyl | 4-I | 195—96 |
| 18b | Na | Ethyl | Ethyl | 4-I | 195—96 |
| 19 | $CH_3$ | Ethyl | Ethyl | 3,4-diCl | 124—26 |
| 20 | $—CH_2CH_2—OH$ | Ethyl | Ethyl | 3,4-diCl | 136—37 |
| 21 | K | Ethyl | Ethyl | 2,5-diCl | 204—06 |
| 21a | Na | Ethyl | Ethyl | 2,5-diCl | 204—06 |
| 22 | K | Ethyl | Ethyl | 2-$CF_3$ | 177—78 |
| 23 | K | Ethyl | Ethyl | 2-Cl,3-$CF_3$ | 190—191 |
| 24a | K | Ethyl | Ethyl | 2-Cl | 195—97 |
| 24b | Na | Ethyl | Ethyl | 2-Cl | 195—97 |
| 25a | K | Ethyl | Ethyl | 3,4-diCl | 185—86 |
| 25b | Na | Ethyl | Ethyl | 3,4-diCl | 185—86 |
| 26 | Na | $CH_3$ | Ethyl | 4-Cl | 223—24 |
| 27a | Na | $CH_3$ | $CH_3$ | 4-Cl | 228—32 |
| 27b | K | $CH_3$ | $CH_3$ | 4-Cl | 228—32 |
| 28 | Na | Ethyl | $CH_3$ | 4-Cl | 189—92 |
| 29 | K | Ethyl | Ethyl | *9 | 198—199°C |

dec = decomposed

* Melting points shown are those of the free-carboxylic acids corresponding to the salts listed. Melting points of Examples 2, 19 and 20 are those of the esters listed.

** The 1-position in this compound is occupied a 4-chloronaphthyl group instead of an optionally substituted phenyl group as shown in the formula above Table I.

TABLE II
Elemental Analysis*
Calculated/Found

| Ex. No. | C | H | H |
|---|---|---|---|
| 1a(1b) | 57.40<br>57.49 | 4.52<br>4.62 | 8.37<br>8.64 |
| 2 | 58.54<br>58.59 | 4.91<br>5.03 | 8.03<br>7.76 |
| 3a(3b) | 63.99<br>63.15 | 5.37<br>5.34 | 9.33<br>8.85 |
| 4a(4b) | 50.67<br>50.95 | 3.99<br>4.02 | 7.39<br>7.30 |
| 5a(5b) | 59.58<br>59.68 | 5.28<br>5.42 | 7.72<br>7.42 |
| 6a(6b) | 50.68<br>50.74 | 3.48<br>4.23 | 7.39<br>7.28 |
| 7a(7b) | 52.05<br>52.30 | 3.82<br>3.32 | 7.59<br>7.60 |
| 8 | 65.84<br>66.06 | 6.13<br>6.25 | 8.54<br>8.60 |
| 9a(9b) | 61.81<br>61.29 | 5.49<br>5.46 | 8.48<br>8.29 |
| 10 | 61.29<br>61.20 | 5.41<br>5.47 | 7.52<br>7.44 |
| 11 | 64.96<br>64.79 | 5.77<br>5.70 | 8.91<br>8.81 |
| 12a(12b) | 55.44<br>55.11 | 4.10<br>4.08 | 7.61<br>7.41 |
| 13a(13b) | 60.38<br>60.16 | 4.75<br>4.75 | 8.80<br>8.61 |
| 14 | 62.76<br>62.70 | 4.64<br>4.68 | 12.93<br>12.79 |
| 15 | 62.78<br>62.48 | 5.85<br>5.84 | 8.14<br>8.11 |
| 16 | 55.65<br>55.84 | 4.37<br>4.32 | 12.17<br>12.13 |
| 17a(17b) | 55.44<br>55.87 | 4.10<br>4.08 | 7.61<br>7.74 |
| 18a(18b) | 45.09<br>45.40 | 3.54<br>3.56 | 6.57<br>6.44 |
| 19 | 53.28<br>53.45 | 4.20<br>4.25 | 7.31<br>7.22 |
| 20 | 52.31<br>52.08 | 4.39<br>4.36 | 6.78<br>6.57 |

TABLE II (Continued)

Elemental Analysis*
Calculated/Found

| Ex. No. | C | H | N |
|---|---|---|---|
| 21a(21b) | 52.05<br>52.47 | 3.82<br>3.84 | 7.59<br>7.73 |
| 22 | 55.44<br>55.56 | 4.10<br>4.06 | 7.61<br>7.51 |
| 23 | 50.70<br>50.72 | 3.50<br>3.36 | 6.95<br>6.92 |
| 24a(24b) | 57.41<br>57.17 | 4.51<br>4.11 | 8.37<br>9.01 |
| 25a(25b) | 52.05<br>51.70 | 3.82<br>3.61 | 7.59<br>7.30 |
| 26 | 56.17<br>55.56 | 4.09<br>3.98 | 8.74<br>8.53 |
| 27a(27b) | 54.82<br>55.69 | 3.62<br>3.73 | 9.14<br>8.77 |
| 28 | 56.17<br>56.18 | 4.09<br>4.11 | 8.74<br>8.64 |
| 29 | 62.43<br>62.15 | 4.45<br>4.46 | 7.28<br>7.08 |

* The elemental analysis data shown are for the free carboxylic acids corresponding to the salts listed in Table I. The elemental analysis data for Examples 2, 19 and 20 are for those esters listed in Table I.

TABLE III
Nuclear Magnetic Resonance (nmr) Data*

| Example No. | Position of Resonances in Parts Per Million Downfield from Tetramethylsilane |
|---|---|
| 1a(1b) | 7.6 (singlet, 4H); 3.0 (quartet, 2H) 2.6 (quartet, 2H); 0.9—1.4 (multiplet, 6H) $CDCl_3$ |
| 2 | 7.6 (singlet, 4H); 4.0 (singlet, 3H); 3.0 (quartet, 2H); 2.6 (quartet, 2H); 0.9—1.4 (multiplet, 6H) $CDCl_3$ |
| 3a(3b) | 7.6 (singlet, 5H); 3.0 (quartet, 2H); 2.6 (quartet, 2H); 0.9—1.4 (multiplet, 6H) $CDCl_3$ |
| 4a(4b) | 7.6 (singlet, 4H); 3.0 (quartet, 2H); 2.6 (quartet, 2H); 0.9—1.4 (multiplet, 6H) $CDCl_3$ |
| 5a(5b) | 7.6 (singlet, 4H); 3.0 (triplet, 2H); 2.60 (triplet, 2H); 1.2—2.17 (multiplet, 5H); 0.53—1.2 (multiplet, 5H) $CDCl_3$ |
| 6a(6b) | 7.5—7.9 (multiplet, 4H); 3.0 (quartet, 2H); 2.5 (quartet, 2H); 1.1 (multiplet, 6H) $CDCl_3$ |
| 7a(7b) | 7.6—7.8 (multiplet, 3H); 2.2—3.3 (multiplet, 4H); 1.2 (multiplet, 6H) $CDCl_3$ |

TABLE III (Continued)
Nuclear Magnetic Resonance (nmr) Data*

| Example | Position of Resonances in Parts Per Million Downfield From Tetramethylsilane |
|---|---|
| 8 | 7.3 (multiplet, 3H): 2.4—3.3 (multiplet, 4H); 2.3 (singlet, 6H); 1.1 (multiplet, 6H) $CDCl_3$ |
| 9a(9b) | 7.2 (AB quartet, 4H); 3.9 (singlet, 3H); 3.0 (quartet, 2H); 2.5 (quartet, 2H); 1.1 (multiplet 6H); $CDCl_3$ |
| 10 | 8.1 (AB quartet, 4H); 4.5 (quartet, 2H); 3.1 (quartet, 2H); 2.6 (quartet, 2H); 0.9—1.7 (multiplet, 9H) $CDCl_3$ |
| 11 | 7.5 (singlet, 4H); 3.7 (quartet, 2H); 3.0 (quartet, 2H); 2.5 (singlet, 3H); 0.9—1.4 (multiplet, 6H) $CDCl_3$ |
| 12a(12b) | 7.8 (multiplet, 4H); 3.0 (quartet, 2H); 2.6 (quartet, 2H); 1.1 (multiplet, 6H) $CDCl_3$ |
| 13a(13b) | 7.1—7.8 (multiplet, 4H); 2.9 (quartet, 2H); 2.5 (quartet, 2H); 1.1 (multiplet, 6H) $CDCl_3$ |
| 14 | 8.1 (multiplet, 4H); 3.0 (quartet, 2H); 2.6 (quartet, 2H); 1.2 (multiplet, 6H) $CDCl_3$ |
| 15 | 7.2 (AB quartet, 4H); 4.1 (quartet, 2H); 3.0 (quartet, 2H); 2.5 (quartet, 2H); 0.9—1.7 (multiplet, 9H) $CDCl_3$ |
| 16 | 8.2 (AB quartet, 4H); 2.0—3.0 (multiplet, 4H); 0.8 (multiplet, 6H) DMSO-$d_6$ |
| 17a(17b) | 7.9 (singlet, 4H); 2.2—3.3 (multiplet, 4H); 1.2 (multiplet, 6H) $CDCl_3$ |
| 18a(18b) | 7.6 (AB quartet, 4H); 2.3—3.2 (multiplet, 4H); 1.1 (multiplet, 6H) $CDCl_3$ |
| 19 | 7.3—7.9 (multiplet, 3H); 4.1 (singlet, 3H); 3.0 (quartet, 2H); 2.6 (quartet, 2H); 1.1 (multiplet, 6H) $CDCl_3$ |
| 20 | 7.3—7.9 (multiplet, 3H); 4.5—4.8 (multiplet, 2H); 3.6—4.2 (multiplet, 2H); 3.0 (quartet, 2H); 2.6 (quartet, 2H); 1.1 (multiplet, 6H) $CDCl_3$ |

* The nmr analysis data shown is that obtained from the analysis of the free carboxylic acids corresponding to the salts listed except for Examples 2, 19 and 20, whose data is for the esters listed.

Typical examples of plant growth regulating compositions incorporating the compounds listed in Table I and similar compounds are as follows:

| Active Ingredient (AI) | Wt% AI | Wt% Surfactant[1] | Carrier/ Diluent | Wt% Carrier/ Diluent |
|---|---|---|---|---|
| (1) Alkali metal salt[2] | 0.1 | 0 | $H_2O$ (uncombined) | 99.9 |
| (2) " | 0.1 | 0.05 | " | 98.85 |
| (3) " | 1.0 | 0 | " | 99.0 |
| (4) " | 1.0 | 0.5 | " | 98.5 |
| (5) " | 5.0 | 2.5 | " | 92.5 |
| (6) " | 50 | 25 | " | 25.0 |
| (7) " | 95 | 2.5 | " | 2.5 |
| (8) " | 99 | 0 | " | 1.0 |
| (9) Free carboxylic acid or ester thereof[3] | 1.0 | 0.5 | water/acetone 1:1 by vol. | 98.5 |
| (10) " | 5.0 | 2.5 | " | 92.5 |
| (11) " | 50 | 25 | " | 25.0 |
| (12) " | 95 | 2.5 | " | 2.5 |

[1] t-Octylphenoxy(polyethyleneoxy)ethanol containing an average of 9—10 ethyleneoxy units.
[2] Each Na or K salt listed in Table I plus the corresponding HCl addition salt.
[3] Each free carboxylic acid of the Na and K salts listed in Table I and the methyl, ethyl n-propyl or 2-hydroxyethyl ester of each of such free carboxylic acids.

As may be required the above formulations are diluted with water to provide ready-for-use compositions for foliar application.

In the biological data and biological test procedures given later units are expressed in U.S. units, the metric equivalents of which are given below, together with other explanatory information.

2 lbs = 0.9 kg
1 gal = 3.8 l
50 gal = 190 l
1 pint = 0.47 l
6 inches = 15.24 cm.
Parts are parts by weight.
Fertilizer (16-25-16) = (16-25-16) by wt. NPK).
1 tsp/gal = 1.3 ml/l
Isotex = benzenehexachloride
Feekes' scale is described by W. Feeks, Vers. 17
Tech. Tarwe Comm, Groningen, pp 560, 561, 1941.
50 gal/A (acre) = 467.5 l/ha.
2 oz/50 gal = 56.7 g/189.25 l
Dose = dose of active ingredient

| lbs/acre | = kg./ha | lbs/acre | = kg./ha |
|---|---|---|---|
| 1/32 | 0.035 | 1/2 | 0.561 |
| 1/16 | 0·07 | 1 | 1.121 |
| 1/8 | 0.140 | 2 | 2.242 |
| 1/4 | 0.280 | 4 | 4.484 |

Chemical Sterilization Activity

Representative compounds listed in Table I above were foliarly applied to spring wheat for inducing male sterility using the following procedures.

The active compound (Na or K salt) to be tested is prepared as a liquid concentrate (abbreviated "LC") consisting of 0.9 kg (2 lbs.) of AI dissolved in 3.8 l (one gallon) of water. To provide test solutions to be applied at various doses, the amounts of LC indicated in the table below are measured out.

| Dose (lbs AI/A) | LC |
|---|---|
| 4 | 2 gal. |
| 2 | 1 gal. |
| 1 | 1/2 gal. |
| 1/2 | 1/4 gal. |
| 1/4 | 1/8 gal. |
| 1/8 | 1/16 gal. |
| 1/16 | 1/32 gal. |
| 1/32 | 1/64 gal. |

To the amount of AI measured out as indicated above, into a 190 l (50 gal.) tank, there is added, first, 0.47 l (1 pint) of the surfactant Triton AG-98 [an octylphenoxy (polyoxyethyleneoxy) ethanol surfactant having an average of 7—8 ethyleneoxy units commercially available from the Rohm and Haas Company, Philadelphia] and then sufficient water to total 190 l (50 gallons). The resulting solution is applied to the test plants at the rate of 467.5 l/ha (50 gal/A).

Carboxylic acid esters were formulated as described above except that the ester derivatives were dissolved in the 190 l (50 gallon) tank in a carrier of water:acetone (1:1 by volume) in place of all water.

Spring wheat (var. Fielder) is planted at the rate of 6 to 8 seeds per 15.24 cm (6 inch) pot containing a sterile medium of 3 parts soil and 1 part humus. The plants are grown under short-day (9 hour) conditions for the first 4 weeks to obtain good vegetative growth before flower initiation. The plants are then moved to long-day (16 hour) conditions which are provided by high intensity lights in the greenhouse. The plants are fertilized at 2, 4 and 8 weeks after planting with a water soluble fertilizer (16—25—16) at the rate of 1.3 ml/l (1 tps/gal) of water, and are frequently sprayed with Isotox for aphid control and dusted with sulfur for powdery mildew control.

Test compounds are foliarly applied to the awned female plants when these plants reach the flag leaf elongation stage (stage 7 on Feekes' scale). All compounds are applied in a carrier volume of 467.5 l/ha (50 gal/A) at the rate of 0.47 l/190 l (1 pint/50 gal). After spike emergence but before anthesis, 4 to 6 spikes per pot are bagged to prevent outcrossing. At the first signs of flower opening, two spikes per pot are cross pollinated, using the approach method, with the awnless male parent. As soon as the seeds become plainly visible, the plants are rated for injury, spike length is measured and seeds per spikelet are counted in both bagged and crossed spikes. Male sterility can then be calculated as percent inhibition of seed set in bagged spikes of treated plants, and female fertility in crossed spikes can be calculated as percent of control seed set. Hybridization is determined by the increase in seed set in the crossed spikes over the bagged spikes.

Percent sterility, percent fertility, and percent hybridization are calculated using the following formulas:

a. % Sterility = $(S_c — S_t)/S_c \times 100$
   $S_c$ = seeds/spikelet in bagged spikes of control plants.
   $S_t$ = seeds/spikelet in bagged spikes of treated plants.

b. % Fertility = $(F_t/F_c) \times 100$
   $F_t$ = seeds/spikelet in approach crossed spikes of treated plants.
   $F_c$ = seeds/spikelet in unbagged spikes of control plants.

c. % Hybridization = $(F_t — S_t)/F_t$

Crop injury, designated by the expression "Injury", means plant spike length inhibition evaluated on a scale of 0—9 where 0 = no spike length inhibition and 9 = dead plants.

Percent height inhibition is calculated according to the following formula:

% Height inhibition = $(H_c{=}H_t/H_c) \times 100$.

$H_c$ = Height of control plants.

$H_t$ = Height of treated plants.

"Dose" means the amount of AI applied expressed in terms of pounds per acre (lb/A).

A dash in the tables means that no determination was made for the given compound at the given rate for the given property.

Tables IV—X summarize typical results obtained in independent sets of evaluations of compounds representative of the invention.

TABLE IV
Biological Date (Greenhouse)
Spring Wheat (var. Fielder)

| Ex. No. | Dose kg/ha (lb/A) | % Male Sterility | % Inhibition | Injury | % Fertilization |
|---|---|---|---|---|---|
| Ib | 0.14 (1/8) | 99 | 25 | 1 | 39 |
| | 0.28 (1/4) | 100 | 25 | 2 | — |
| | 0.561 (1/2) | 100 | 30 | 4 | — |
| | 1.121 (1) | 100 | 50 | 9 | — |

TABLE V
Biological Data
Winter Wheat Var Newton (Field Test)

| Ex. No. | Dose kg/ha (lb/A) | % Male Sterility | Injury (0—9) | Yield % of Control |
|---|---|---|---|---|
| Ib | 0.14 (1/8) | 27 | 0 | 88 |
| | 0.28 (1/4) | 76 | 0.7 | 71 |
| | 0.561 (1/2) | 97 | 4.3 | 35 |
| | 1.121 (1) | 98 | 6.3 | 19 |

TABLE VI
Biological Data
Winter Barley Var Henry (Field Test)

| Ex. No. | Dose kg/ha (lb/A) | % Male Sterility | Injury (0—9) | Yield % of Control |
|---|---|---|---|---|
| 1a | 0.561 (1/2) | 99 | 0 | 70 |
| | 1.121 (1) | 100 | 1.5 | 63 |
| | 2.242 (2) | 91 | 1.0 | 64 |
| | 4.484 (4) | 100 | 2.0 | 65 |

TABLE VII
Biological Data (Greenhouse)
Spring Wheat (var. Fielder)

| Ex. No. | Dose kg/ha (lb/A) | % Male Sterility | % Inhibition | Injury | % Fertilization |
|---|---|---|---|---|---|
| 2 | 0.035 (1/32) | 88 | 20 | 2 | 91 |
| | 0.07 (1/16) | 95 | 30 | 2 | — |
| | 0.14 (1/8) | 100 | 50 | 3 | — |
| | 0.28 (1/4) | 100 | 50 | 3 | — |
| | 0.561 (1/2) | 100 | 50 | 4 | — |
| 3a. | 0.035 (1/32) | 0 | 0 | 0 | — |
| | 0.07 (1/16) | 19 | 0 | 0 | — |
| | 0.14 (1/8) | 91 | 0 | 0 | 100 |
| | 0.28 (1/4) | 100 | 0 | 0.5 | 74 |
| | 0.561 (1/2) | 100 | 10 | 0.5 | 34 |
| 4a | 0.035 (1/32) | 0 | 0 | 0 | — |
| | 0.07 (1/16) | 29 | 0 | 0 | — |
| | 0.14 (1/8) | 81 | 0 | 0 | 100 |
| | 0.28 (1/4) | 100 | 20 | 0 | 93 |
| | 0.561 (1/2) | 100 | 30 | 1 | — |
| 1b | 0.035 (1/32) | 9 | 0 | 0 | — |
| | 0.07 (1/16) | 57 | 0 | 0 | — |
| | 0.14 (1/8) | 99 | 0 | 0 | 96 |
| | 0.28 (1/4) | 100 | 30 | 1 | 95 |
| | 0.561 (1/2) | 100 | 40 | 2 | — |

TABLE VIII
Biological Data (Greenhouse)
Spring Wheat (var. Fielder)

| Ex. No. | Dose kg/ha (lb/A) | % Male Sterility | % Inhibition | Injury | % Fertilization |
|---|---|---|---|---|---|
| 1b | 0.07 (1/16) | 90 | 0 | 0 | 94 |
| | 0.14 (1/8) | 100 | 5 | 2 | — |
| | 0.28 (1/4) | 100 | 20 | 2 | — |
| | 0.561 (1/2) | 100 | 25 | 3 | — |
| 7a | 0.14 (1/8) | 20 | 0 | 0 | — |
| | 0.28 (1/4) | 74 | 0 | 0 | — |
| | 0.561 (1/2) | 100 | 5 | 1 | 51 |
| | 1.121 (1) | 100 | 10 | 2 | 12 |
| 24a | 0.14 (1/8) | 0 | 0 | 0 | — |
| | 0.28 (1/4) | 55 | 0 | 0 | — |
| | 0.561 (1/2) | 100 | 0 | 0 | — |
| | 1.121 (1) | 100 | 0 | 0 | 48 |
| 12a | 0.14 (1/8) | 100 | 0 | 0.5 | 87 |
| | 0.28 (1/4) | 100 | 15 | 2 | 28 |
| | 0.561 (1/2) | 100 | 25 | 3 | — |
| | 1.121 (1) | 100 | 30 | 4 | — |
| 17a | 0.14 (1/8) | 100 | 0 | 0 | — |
| | 0.28 (1/4) | 100 | 10 | 0 | 13 |
| | 0.561 (1/2) | 100 | 20 | 1 | 38 |
| | 1.121 (1) | 100 | 30 | 1 | — |

TABLE IX
Biological Data (Greenhouse)
Spring Wheat (var. Fielder)

| Ex. No. | Dose kg/ha (lb/A) | % Male Sterility | % Inhibition | Injury | % Fertilization |
|---|---|---|---|---|---|
| 1b | 0.07 (1/16) | 100 | 0 | 0.5 | 83 |
| | 0.14 (1/8) | 100 | 10 | 2 | — |
| | 0.28 (1/4) | 100 | 30 | 3 | — |
| | 0.561 (1/2) | 100 | 40 | 5 | — |
| modi-fied* | 0.14 (1/8) | 100 | 20 | 3 | — |
| | 0.28 (1/4) | 100 | 30 | 4 | — |
| | 0.561 (1/2) | 100 | 40 | 5 | — |
| | 1.121 (1) | 100 | 60 | 6 | — |
| 20 | 0.14 (1/8 | 93 | 10 | 2 | 92 |
| | 0.28 (1/4) | 100 | 30 | 3 | — |
| | 0.561 (1/2) | 100 | 40 | 4 | — |
| | 1.121 (1) | 100 | 50 | 6 | — |
| 19 | 0.14 (1/8) | 100 | 30 | 1 | — |
| | 0.28 (1/4) | 100 | 30 | 2 | — |
| | 0.561 (1/2) | 98 | 40 | 3 | — |
| | 1.121 (1) | 100 | 40 | 4 | — |
| 25b | 0.14 (1/8) | 100 | 30 | 3 | — |
| | 0.28 (1/4) | 100 | 40 | 4 | — |
| | 0.561 (1/2) | 100 | 50 | 5 | — |
| | 1.121 (1) | 100 | 80 | 8 | — |

* same compound as that of Example 20 except that X = 4-Cl.

TABLE X
Biological Data (Greenhouse
Spring Wheat (var. Fielder)

| Ex. No. | Dose kg/ha (lb/A) | % Male Sterility | % Inhibition | Injury |
|---|---|---|---|---|
| 1b | 0.07 (1/16) | 100 | 10 | 1 |
| | 0.14 (1/8) | 100 | 20 | 1 |
| | 0.28 (1/4) | 100 | 20 | 2 |
| | 0.561 (1/2) | 100 | 20 | 4 |
| 6a | 0.14 (1/8) | 100 | 0 | 0.5 |
| | 0.28 (1/4) | 100 | 0 | 0.5 |
| | 0.561 (1/2) | 100 | 10 | 2 |
| | 1.121 (1) | 100 | 20 | 3 |
| 18a | 0.14 (1/8) | 100 | 0 | 0 |
| | 0.28 (1/4) | 100 | 10 | 0.5 |
| | 0.561 (1/2) | 100 | 20 | 1 |
| | 1.121 (1) | 100 | 25 | 3 |
| 9a | 0.14 (1/8) | 100 | 0 | 0 |
| | 0.28 (1/4) | 100 | 0 | 0 |
| | 0.561 (1/2) | 100 | 10 | 0.5 |
| | 1.121 (1) | 99 | 10 | 0.5 |
| 13a | 0.14 (1/8) | 100 | 15 | 3 |
| | 0.28 (1/4) | 100 | 20 | 4 |
| | 0.561 (1/2) | 100 | 25 | 5 |
| | 1.121 (1) | 100 | 25 | 6 |

Similar results to those given in Tables IV—X are obtained by (a) replacing the Na salts specified in the Tables with the corresponding K salts and vice versa, (b) using the HCl, HBr, $H_2SO_4$ and $HNO_3$ acid addition salts of each of the compounds specified in the Tables and (c) replacing the carboxylic acid ester group in each of the esters specified with a different ester group selected from $CO_2CH_3$, $CO_2C_2H_5$, $CO_2CH_2OH$, $CO_2CH(OH)CH_3$ and $CO_2CH_2CH_2OH$.

**Claims for the Contracting States: BE CH DE FR GB IT LI NL SE**

1. A compound having the formula:

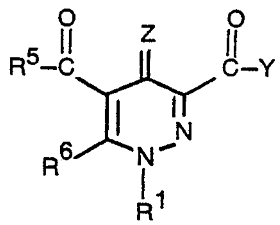

(I)

wherein:

$R^1$ is phenyl or naphthyl each optionally substituted with up to three of the same or different substituents selected from halogen, $(C_1—C_4)$alkyl, $(C_1—C_4)$alkoxy, ethoxycarbonyl, trihalomethyl, nitro and cyano;

Y is $Z^1R^3$ or $NR^7R^8$ wherein $Z^1$ is O or S; $R^3$ is hydrogen, $(C_1—C_6)$alkyl, 2-hydroxyethyl, $(C_1—C_4)$alkoxy$(C_1—C_4)$alkyl, $(C_3—C_6)$cycloalkyl, $(C_3—C_6)$cycloalkyl$(C_1—C_4)$alkyl, halo$(C_1—C_3)$alkyl containing up to three halogen atoms, phenyl or benzyl; and $R^7$ and $R^8$ are (a) the same or different and are selected from, hydrogen, $(C_1—C_4)$alkyl, $(C_1—C_4)$alkyl substituted with a carboxyl group and $(C_1—C_4)$alkoxy substituted with a carboxyl group or (b) are joined together and form an alkylene group having up to 5 carbon atoms;

Z is O or S;

$R^5$ is $(C_1—C_4)$alkyl or phenyl$(C_1—C_4)$alkyl and

$R^6$ is $(C_1—C_4)$alkyl or phenyl$(C_1—C_4)$alkyl; and agronomically acceptable derivatives of compounds of Formula I being metal and ammonium salts, acid addition salts and quaternary ammonium addition salts.

2. A compound as claimed in Claim 1, wherein

$R^1$ is phenyl or phenyl substituted with up to three substituents selected from halogen, $(C_1—C_4)$alkyl, $(C_1—C_4)$alkoxy, trihalomethyl, nitro and cyano;

Y is $Z^1R^3$ or $NR^7R^8$ wherein $Z^1$ is 0, $R^3$ is $(C_1—C_4)$alkyl, an alkali metal cation, an alkaline earth metal cation, or ammonium or alkyl-substituted ammonium; and $R^7$ and $R^8$ are each $(C_1—C_4)$alkyl, or $R^7$ is hydrogen and $R^8$ is $—CH_2C(O)OR^9$ wherein $R^9$ is an alkali metal cation or methyl;

$R^5$ is $(C_1—C_4)$alkyl; and

$R^6$ is $(C_1—C_4)$alkyl.

3. A compound as claimed in Claim 1, wherein $R^1$ is phenyl optionally substituted with one substituent which is Cl, Br, I, F, CN, $CF_3$, methoxy or ethoxy or two substituents which are both Cl or are one each of Cl and $CF_3$.

4. A compound as claimed in Claim 1, wherein:

$R^1$ is phenyl, 4-chlorophenyl, 4-bromophenyl, 4-fluorophenyl, 3,4-dichlorophenyl, 4-trifluoromethylphenyl or 4-iodophenyl;

Y is $Z^1R^3$ wherein $Z^1$ is O and $R^3$ is $(C_1—C_4)$alkyl or an alkali metal cation;

$R^5$ is $(C_2—C_4)$alkyl; and

$R^6$ is $(C_2—C_4)$alkyl.

5. A compound as claimed in Claim 1, wherein $R^1$ is phenyl, Y is ONa or OK and $R^5$ and $R^6$ are both $C_2H_5$.

6. A compound as claimed in Claim 1, wherein $R^1$ is 4-bromophenyl or 3-bromophenyl, Y is ONa or OK and $R^5$ and $R^6$ are both $C_2H_5$.

7. A compound as claimed in Claim 1, wherein $R^1$ is 4-chlorophenyl, Y is ONa or OK and $R^5$ and $R^6$ are both $C_3H_7$.

8. A compound as claimed in Claim 1, wherein $R^1$ is 2-chlorophenyl, 4-chlorophenyl or 3,4-dichlorophenyl, Y is ONa, OK, $OCH_3$ or $OCH_2CH_2OH$ and $R^5$ and $R^6$ are both $C_2H_5$.

9. A compound as claimed in Claim 1, wherein $R^1$ is 4-fluorophenyl, Y is OK or ONa and $R^5$ and $R^6$ are both $C_2H_5$.

10. A compound as claimed in Claim 1, wherein $R^1$ is 4-iodophenyl, Y is OK or ONa, and $R^5$ and $R^6$ are both $C_2H_5$.

11. A compound as claimed in Claim 1, wherein $R^1$ is 3-trifluoromethylphenyl, 4-trifluoromethylphenyl or 3,5-dichlorophenyl, Y is OK or ONa and $R^5$ and $R^6$ are both $C_2H_5$.

12. A compound as claimed in Claim 1, wherein $R^1$ is 4-methoxyphenyl, Y is OK or ONa and $R^5$ and $R^6$ are both $C_2H_5$.

13. A compound as claimed in Claim 1, wherein $R^1$ is 2,4-dichlorophenyl, Y is OK or ONa and $R^5$ and $R^6$ are both $C_2H_5$.

14. A compound as claimed in Claim 1, wherein $R^1$ is 4-chlorophenyl, Y is OK or ONa and $R^5$ and $R^6$ are both $CH_3$.

15. A method of regulating the growth of a gramineous crop plant which comprises applying to the growing plant, or to seeds thereof, a growth regulating amount of one or more compounds as claimed in any one of Claims 1—14.

16. A method as claimed in Claim 15 as applied to the production of hybrid seed by using at least one growth regulating compound which induces male sterility without destroying female fertility.

17. A method as claimed in Claim 16, wherein the plant is maize, barley, wheat, sorghum or a forage crop plant.

18. A method as claimed in Claim 16 or 17 which includes the step of treating a male/female parent of the gramineous plant prior to meiosis with the growth regulating compound.

19. A plant growth regulating composition which comprises a compound as claimed in any one of Claims 1—14 and an agronomically acceptable carrier or diluent therefor.

**Claims for the Contracting State: AT**

1. A plant growth regulating composition containing, as active ingredient, a substituted pyridazine or an agronomically acceptable derivative thereof which is a metal or ammonium salt, an acid addition salt or a quaternary ammonium addition salt, the composition also containing an agronomically acceptable diluent or carrier for the active ingredient, characterized in that the substituted pyridazine is of the formula:

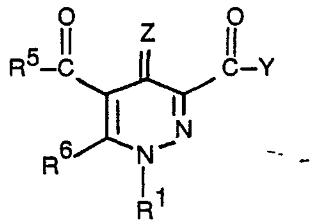

(I)

wherein:

$R^1$ is phenyl or naphthyl each optionally substituted with up to three of the same or different substituents selected from halogen, $(C_1$—$C_4)$alkyl, $(C_1$—$C_4)$alkoxy, ethoxycarbonyl, trihalomethyl, nitro and cyano;

Y is $Z^1R^3$ or $NR^7R^8$ wherein $Z^1$ is O or S; $R^3$ is hydrogen, $(C_1$—$C_6)$alkyl, 2-hydroxyethyl, $(C_1$—$C_4)$alkoxy$(C_1$—$C_4)$alkyl, $(C_3$—$C_6)$cycloalkyl, $(C_3$—$C_6)$cycloalkyl$(C_1$—$C_4)$alkyl, halo$(C_1$—$C_3)$alkyl containing up to three halogen atoms, phenyl or benzyl; and $R^7$ and $R^8$ (a) are the same or different and are selected from, hydrogen, $(C_1$—$C_4)$alkyl, $(C_1$—$C_4)$alkyl substituted with a carboxyl group and $(C_1$—$C_4)$alkoxy substituted with a carboxyl group or (b) are joined together and form an alkylene group having up to 5 carbon atoms;

Z is O or S;

$R^5$ is $(C_1$—$C_4)$alkyl or phenyl$(C_1$—$C_4)$ alkyl); and

$R^6$ is $(C_1$—$C_4)$alkyl or phenyl$(C_1$—$C_4)$alkyl.

2. A composition as claimed in Claim 1, wherein

$R^1$ is phenyl or phenyl substituted with up to three substituents selected from halogen, $(C_1$—$C_4)$alkyl, $(C_1$—$C_4)$alkoxy, trihalomethyl, nitro and cyano;

Y is $Z^1R^3$ or $NR^7R^8$ wherein $Z^1$ is 0, $R^3$ is $(C_1$—$C_4)$alkyl, an alkali metal cation, an alkaline earth metal cation, or ammonium or alkyl-substituted ammonium; and $R^7$ and $R^8$ are each $(C_1$—$C_4)$alkyl, or $R^7$ is hydrogen and $R^8$ is —$CH_2C(O)OR^9$ wherein $R^9$ is an alkali metal cation or methyl;

$R^5$ is $(C_1$—$C_4)$alkyl; and

$R^6$ is $(C_1$—$C_4)$alkyl.

3. A composition as claimed in Claim 1, wherein $R^1$ is phenyl optionally substituted with one substituent which is Cl, Br, I, F, CN, $CF_3$, methoxy or ethoxy or two substituents which are both Cl or are one each of Cl and $CF_3$.

4. A composition as claimed in Claim 1, wherein:

$R^1$ is phenyl, 4-chlorophenyl, 4-bromophenyl, 4-fluorophenyl, 3,4-dichlorophenyl, 4-trifluoromethylphenyl or 4-iodophenyl;

Y is $Z^1R^3$ wherein $Z^1$ is O and $R^3$ is $(C_1$—$C_4)$alkyl or an alkali metal cation;

$R^5$ is $(C_2$—$C_4)$alkyl; and

$R^6$ is $(C_2$—$C_4)$alkyl.

5. A composition as claimed in Claim 1, wherein $R^1$ is phenyl, Y is ONa or OK and $R^5$ and $R^6$ are both $C_2H_5$.

6. A composition as claimed in Claim 1, wherein $R^1$ is 4-bromophenyl or 3-bromophenyl, Y is ONa or OK and $R^5$ and $R^6$ are both $C_2H_5$.

7. A composition as claimed in Claim 1, wherein $R^1$ is 4-chlorophenyl, Y is ONa or OK and $R^5$ and $R^6$ are both $C_3H_7$.

8. A composition as claimed in Claim 1, wherein $R^1$ is 2-chlorophenyl, 4-chlorophenyl or 3,4-dichlorophenyl, Y is ONa, OK, $OCH_3$ or $OCH_2CH_2OH$ and $R^5$ and $R^6$ are both $C_2H_5$.

9. A composition as claimed in Claim 1, wherein $R^1$ is 4-fluorophenyl, Y is OK or ONa and $R^5$ and $R^6$ are both $C_2H_5$.

10. A composition as claimed in Claim 1, wherein $R^1$ is 4-iodophenyl, Y is OK or ONa, and $R^5$ and $R^6$ are both $C_2H_5$.

11. A composition as claimed in Claim 1, wherein $R^1$ is 3-trifluoromethylphenyl, 4-trifluoromethylphenyl or 3,5-dichlorophenyl, Y is OK or ONa and $R^5$ and $R^6$ are both $C_2H_5$.

12. A composition as claimed in Claim 1, wherein $R^1$ is 4-methoxyphenyl, Y is OK or ONa and $R^5$ and $R^6$ are both $C_2H_5$.

13. A composition as claimed in Claim 1, wherein $R^1$ is 2,4-dichlorophenyl, Y is OK or ONa and $R^5$ and $R^6$ are both $C_2H_5$.

14. A composition as claimed in Claim 1, wherein $R^1$ is 4-chlorophenyl, Y is OK or ONa and $R^5$ and $R^6$ are both $CH_3$.

15. A method of regulating the growth of a gramineous crop plant which comprises applying to the growing plant, or to seeds thereof, a growth regulating amount of a composition as claimed in any one of Claims 1—14.

16. A method as claimed in Claim 15 as applied to the production of hybrid seed by using at least one growth regulating compound which induces male sterility without destroying female fertility.

17. A method as claimed in Claim 16, wherein the plant is maize, barley, wheat, sorghum or a forage crop plant.

18. A method as claimed in Claim 16 or 17 which comprises treating a male/female parent of the gramineous plant prior to meiosis with the growth regulating compound.

**Patentansprüche für die Vertragsstaaten: BE CH DE FR GB IT LI NL SE**

1. Verbindung der Formel

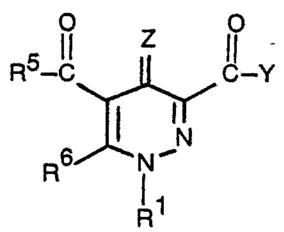

(I)

worin $R^1$

Phenyl oder Naphthyl ist, jeweils gegebenenfalls substituiert mit bis zur drei gleichen oder verschiedenen Substituenten, ausgewählt aus Halogen, $(C_1—C_4)$Alkyl, $(C_1—C_4)$Alkoxy, Ethoxycarbonyl, Trihalogenmethyl, Nitro und Cyano,

Y $Z^1R^3$ oder $NR^7R^8$ ist, worin $Z^1$ O oder S ist, $R^3$ Wasserstoff, $(C_1—C_6)$Alkyl, 2-Hydroxyethyl, $(C_1—C_4)$Alkoxy$(C_1—C_4)$Alkyl, $(C_3—C_6)$Cycloalkyl, $(C_3—C_6)$Cycloalkyl$(C_1—C_4)$Alkyl, Halogen$(C_1—C_3)$Alkyl, enthaltend bis zu drei Halogenatome, Phenyl oder Benzyl ist, und $R^7$ und $R^8$ (a) gleich oder verschieden sind und ausgewählt sind aus Wasserstoff, $(C_1—C_4)$Alkyl, $(C_1—C_4)$Alkyl, substituiert mit einer Carboxylgruppe, und $(C_1—C_4)$Alkoxy, substituiert mit einer Carboxylgruppe, oder (b) miteinander verbunden sind und eine Alkylengruppe bis zu 5 Kohlenstoffatomen bilden,

Z O oder S ist,

$R^5$ $(C_1—C_4)$Alkyl oder Phenyl$(C_1—C_4)$Alkyl ist und

$R^6$ $(C_1—C_4)$Alkyl oder Phenyl$(C_1—C_4)$Alkyl ist,

wobei für landwirtschaftliche Zwecke verträgliche Derivate von Verbindungen der Formel I Metall- und Ammoniumsalze, Säureadditionssalze und quaternäre Ammoniumadditionssalze sind.

2. Verbindung nach Anspruch 1, wobei

$R^1$ Phenyl oder Phenyl, substituiert mit bis zu drei Substituenten ist, ausgewählt aus Halogen, $(C_1—C_4)$Alkyl, $(C_1—C_4)$Alkoxy, Trihalogenmethyl, Nitro und Cyano,

Y $Z^1R^3$ oder $NR^7R^8$ ist, worin $Z^1$ O is, $R^3$ $(C_1—C_4)$Alkyl, ein Alkalimetallkation, ein Erdalkalimetallkation oder Ammonium oder Alkyl-substituiertes Ammonium ist und $R^7$ und $R^8$ jeweils $(C_1—C_4)$Alkyl sind, oder $R^7$ Wasserstoff ist und $R^8$ $—CH_2C(O)OR^9$ ist, worin $R^9$ ein Alkalimetallkation oder Methyl ist,

$R^5$ $(C_1—C_4)$Alkyl ist und

$R^6$ $(C_1—C_4)$Alkyl ist.

3. Verbindung nach Anspruch 1, worin $R^1$ Phenyl ist, gegebenenfalls substituiert mit einem Substituenten, der aus Cl, Br, J, F, CN, $CF_3$, Methoxy oder Ethoxy besteht, oder mit zwei Substituenten, die jeweils Cl sind, oder von denen jeweils einer aus Cl und $CF_3$ besteht.

4. Verbindung nach Anspruch 1, worin:

$R^1$ Phenyl, 4-Chlorphenyl, 4-Bromphenyl, 4-Fluorphenyl, 3,4-Dichlorphenyl, 4-Trifluormethylphenyl oder 4-Jodphenyl ist,

Y $Z^1R^3$ ist, worin $Z^1$ O ist und $R^3$ $(C_1—C_4)$Alkyl oder ein Alkalimetallkation ist,

$R^5$ $(C_2—C_4)$Alkyl ist und

$R^6$ $(C_2—C_4)$Alkyl ist.

5. Verbindung nach Anspruch 1, worin $R^1$ Phenyl ist, Y ONa oder OK ist und $R^5$ und $R^6$ beide $C_2H_5$ sind.

6. Verbindung nach Anspruch 1, worin $R^1$ 4-Bromphenyl oder 3-Bromphenyl ist, Y ONa oder OK ist und $R^5$ und $R^6$ beide $C_2H_5$ sind.

7. Verbindung nach Anspruch 1, worin $R^1$ 4-Chlorphenyl ist, Y ONa oder OK ist und $R^5$ und $R^6$ beide $C_3H_7$ sind.

8. Verbindung nach Anspruch 1, worin $R^1$ 2-Chlorphenyl, 4-Chlorphenyl oder 3,4-Dichlorphenyl ist, Y ONa, OK, $OCH_3$ oder $OCH_2CH_2OH$ ist und $R^5$ und $R^6$ beide $C_2H_5$ sind.

9. Verbindung nach Anspruch 1, worin $R^1$ 4-Fluorphenyl ist, Y OK oder ONa ist und $R^5$ und $R^6$ beide $C_2H_5$ sind.

10. Verbindung nach Anspruch 1, worin $R^1$ 4-Jodphenyl ist, Y OK oder ONa ist und $R^5$ und $R^6$ beide $C_2H_5$ sind.

11. Verbindung nach Anspruch 1, worin $R^1$ 3-Trifluormethylphenyl, 4-Trifluormethylphenyl oder 3,5-Dichlorphenyl ist, Y OK oder ONa ist und $R^5$ und $R^6$ beide $C_2H_5$ sind.

12. Verbindung nach Anspruch 1, worin $R^1$ 4-Methoxyphenyl ist, Y OK oder ONa ist und $R^5$ und $R^6$ beide $C_2H_5$ sind.

13. Verbindung nach Anspruch 1, worin $R^1$ 2,4-Dichlorphenyl ist, Y OK oder ONa ist und $R^5$ und $R^6$ beide $C_2H_5$ sind.

14. Verbindung nach Anspruch 1, worin $R^1$ 4-Chlorphenyl ist, Y OK oder ONa ist und $R^5$ und $R^6$ beide $CH_3$ sind.

15. Verfahren zur Regulierung des Wachstums einer grasartigen Nutzpflanze, dadurch gekennzeichnet, daß auf die wachsende Pflanze oder auf ihren Samen eine wachstumsregulierende Menge einer oder mehrer Verbindungen gemäß einem der Ansprüche 1 bis 14 aufgebracht wird.

16. Verfahren nach Anspruch 15, angewendet auf die Erzeugung einer Hybridsaat unter Verwendung wenigstens einer wachstumsregulierenden Verbindung, die eine männliche Sterilität induziert, ohne dabei die weibliche Fertilität zu zerstören.

17. Verfahren nach Anspruch 16, wobei die Pflanze aus Mais, Gerste, Weizen, Sorghum oder einer Futternutzpflanze besteht.

18. Verfahren nach Anspruch 16 oder 17, wobei die Stufe einer Behandlung eines männlichen/weiblichen Elternteils der grasartigen Pflanze vor der Meiosis mit der wachstumsregulierenden Verbindungen vorgesehen ist.

19. Pflanzenwachstumsregulierende Zubereitung aus einer Verbindung gemäß einem der Anspruch 1 bis 14 und einem für landwirtschaftliche Zwecke verträglichen Träger oder Verdünnungsmittel dafür.

**Patentansprüche für den Vertragsstaat: AT**

1. Pflanzenwachstumsregulierende Mittel, enthaltend als Wirkstoff ein substituiertes Pyridazin oder ein für landwirtschaftliche Zwecke verträgliches Derivat davon, das ein Metall- oder Ammoniumsalz, ein Säureadditionssalz oder ein quaternäres Ammoniumadditionssalz ist, wobei das Mittel auch ein für landwirtschaftliche Zwecke verträgliches Verdünnungsmittel oder einen für landwirtschaftliche Zwecke verträglichen Träger für den Wirkstoff enthält, dadurch gekennzeichnet, daß das substituiert Pyridazin der Formel

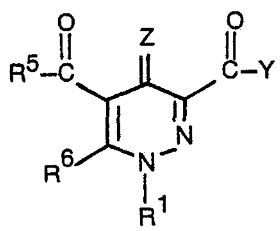

(I)

entspricht worin bedeuten:

$R^1$ Phenyl oder Naphthyl, jeweils gegebenenfalls substituiert mit bis zu drei gleichen oder verschiedenen Substituenten, ausgewählt aus Halogen, $(C_1—C_4)$Alkyl, $(C_1—C_4)$Alkoxy, Ethoxycarbonyl, Trihalogenmethyl, Nitro und Cyano,

Y $Z^1R^3$ oder $NR^7R^8$ ist, worin $Z^1$ für O oder S steht, $R^3$ Wasserstoff, $(C_1—C_6)$Alkyl, 2-Hydroxyethyl, $(C_1—C_4)$Alkoxy$(C_1—C_4)$Alkyl, $(C_3—C_6)$Cycloalkyl, $(C_3—C_6)$Cycloalkyl$(C_1—C_4)$Alkyl, Halogen$(C_1—C_3)$Alkyl, enthaltend bis zu 3 Halogenatome, Phenyl oder Benzoyl ist, und $R^7$ und $R^8$ (a) gleich oder verschieden sind und ausgewählt sind aus Wasserstoff, $(C_1—C_4)$Alkyl, $(C_1—C_4)$Alkyl, substituiert mit einer Carboxylgruppe, und $(C_1—C_4)$Alkoxy, substituiert mit einer Carboxylgruppe, oder (b) miteinander verbunden sind und eine Alkylengruppe mit bis zu 5 Kohlenstoffatomen bilden,

Z O oder S ist,

$R^5$ $(C_1—C_4)$Alkyl oder Phenyl$(C_1—C_4)$Alkyl ist und

$R^6$ $(C_1—C_4)$Alkyl oder Phenyl$(C_1—C_4)$Alkyl ist.

2. Mittel nach Anspruch 1, dadurch gekennzeichnet, daß

$R^1$ Phenyl oder Phenyl, substituiert mit bis zu drei Substituenten, ausgewählt aus Halogen, $(C_1—C_4)$Alkyl, $(C_1—C_4)$Alkoxy, Trihalogenmethyl, Nitro und Cyano, ist,

Y $Z^1R^3$ oder $NR^7R^8$ ist, worin $Z^1$ O is, $R^3$ $(C_1—C_4)$Alkyl, ein Alkalimetallkation, ein Erdalkalimetallkation oder Ammonium oder Alkyl-substituiertes Ammonium ist, und $R^7$ und $R^8$ jeweils $(C_1—C_4)$Alkyl sind, oder $R^7$ Wasserstoff ist und $R^8$ $—CH_2C(O)OR^9$ ist, worin $R^9$ ein Alkalimetallkation oder Methyl ist,

$R^5$ $(C_1—C_4)$Alkyl ist und
$R^6$ $(C_1—C_4)$Alkyl ist.

3. Mittel nach Anspruch 1, dadurch gekennzeichnet, daß $R^1$ Phenyl ist, gegebenenfalls substituiert mit einem Substituenten, der aus Cl, Br, J, F, CN, $CF_3$, Methoxy oder Ethoxy besteht, oder mit zwei Substituenten, die jeweils beide Cl sind oder einer aus Cl und der andere aus $ZF_3$ besteht.

4. Mittel nach Anspruch 1, dadurch gekennzeichnet, daß
$R^1$ Phenyl, 4-Chlorphenyl, 4-Bromphenyl, 4-Fluorphenyl, 3,4-Dichlorphenyl, 4-Trifluormethylphenyl oder 4-Jodphenyl ist,
Y $Z^1R^3$ ist, worin $Z^1$ O ist und $R^3$ $(C_1—C_4)$Alkyl oder ein Alkalimetallkation ist,
$R^5$ $(C_2—C_4)$Alkyl ist und
$R^6$ $(C_2—C_4)$Alkyl bedeutet.

5. Mittel nach Anspruch 1, dadurch gekennzeichnet, daß $R^1$ Phenyl ist, Y ONa oder OK ist und $R^5$ und $R^6$ jeweils $C_2H_5$ sind.

6. Mittel nach Anspruch 1, dadurch gekennzeichnet, daß $R^1$ 4-Bromphenyl oder 3-Bromphenyl ist, Y ONa oder OK ist und $R^5$ und $R^6$ jeweils $C_2H_5$ sind.

7. Mittel nach Anspruch 1, dadurch gekennzeichnet, daß $R^1$ 4-Chlorphenyl ist, Y ONa oder OK bedeutet und $R^5$ und $R^6$ jeweils $C_3H_7$ sind.

8. Mittel nach Anspruch 1, dadurch gekennzeichnet, daß $R^1$ 2-Chlorphenyl, 4-Chlorphenyl oder 3,4-Dichlorphenyl ist, Y ONa, OK, $OCH_3$ oder $OCH_2CH_2OH$ ist und $R^5$ und $R^6$ jeweils $C_2H_5$ sind.

9. Mittel nach Anspruch 1, dadurch gekennzeichnet, daß $R^1$ 4-Fluorphenyl ist, Y OK oder ONa ist und $R^5$ und $R^6$ jeweils $C_2H_5$ sind.

10. Mittel nach Anspruch 1, dadurch gekennzeichnet, daß $R^1$ 4-Jodphenyl ist, Y OK oder ONa ist und $R^5$ und $R^6$ jeweils $C_2H_5$ sind.

11. Mittel nach Anspruch 1, dadurch gekennzeichnet, daß $R^1$ 3-Trifluormethylphenyl, 4-Trifluormethylphenyl oder 3,5-Dichlorphenyl ist, Y OK oder ONa ist und $R^5$ und $R^6$ jeweils $C_2H_5$ sind.

12. Mittel nach Anspruch 1, dadurch gekennzeichnet, daß $R^1$ 4-Methoxyphenyl ist, Y OK oder ONa ist und $R^5$ und $R^6$ jeweils $C_2H_5$ sind.

13. Mittel nach Anspruch 1, dadurch gekennzeichnet, daß $R^1$ 2,4-Dichlorphenyl ist, Y OK oder ONa ist und $R^5$ und $R^6$ jeweils $C_2H_5$ sind.

14. Mittel nach Anspruch 1, dadurch gekennzeichnet, daß $R^1$ 4-Chlorphenyl ist, Y OK oder ONa ist und $R^5$ und $R^6$ jeweils $CH_3$ sind.

15. Verfahren zur Regulierung des Wachstums einer grasartigen Nutzpflanze, dadurch gekennzeichnet, daß auf die wachsende Pflanze oder auf ihre Saat eine wachstumsregulierende Menge eines Mittels gemäß einem der Ansprüche 1 bis 14 aufgebracht wird.

16. Verfahren nach Anspruch 15, angewendet auf die Erzeugung einer Hybridsaat unter Verwendung wenigstens einer wachstumsregulierenden Verbindung, die eine männliche Sterilität induziert, ohne dabei die weibliche Fertilität zu zerstören.

17. Verfahren nach Anspruch 16, dadurch gekennzeichnet die Pflanze aus Mais, Gerste, Weizen, Sorghum oder einer Futternutzpflanze besteht.

18. Verfahren nach Anspruch 16 oder 17, dadurch gekennzeichnet, daß ein männlich/weiblicher Elternteil der grasartigen Pflanze vor der Meiosis mit der wachstumsregulierenden Verbindung behandelt wird.

**Revendications pour les Etats contractants: BE CH DE FR GB IT LI NL SE**

1. Un composé répondant à la formule:

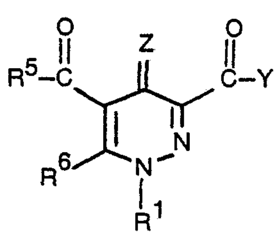

(I)

dans laquelle:

$R^1$ est un phényle ou un naphtyle substitués chacun éventuellement par jusqu'à trois substituants semblables ou différents choisis parmi un halogène, un alcoyle $(C_1—C_4)$, un alcoxy $(C_1—C_4)$, un éthoxycarbonyle, un trihalogénométhyle, un nitro et un cyano;

Y est $Z^1R^3$ ou $NR^7R^8$ où $Z^1$ est O ou S; $R^3$ est un hydrogène un alcoyle $(C_1—C_6)$, un 2-hydroxyéthyle, un alcoxy $(C_1—C_4)$ alcoyle $(C_1—C_4)$, un cycloalcoyle $(C_3—C_6)$, un cycloalcoyl $(C_3—C_6)$ alcoyle $(C_1—C_4)$, un halogénoalcoyle $(C_1—C_3)$ contenant jusqu'à 3 atomes d'halogène, un phényle ou un benzyle; et $R^7$ et $R^8$ sont (a) semblables ou différents et choisis parmi un hydrogène, un alcoyle $(C_1—C_4)$, un alcoyle $(C_1—C_4)$ substitué par un groupe carboxyle et un alcoxy $(C_1—C_4)$ substitué par un groupe carboxyle ou (b) sont réunis et forment un groupe alcoylène ayant jusqu'à 5 atomes de carbone;

Z est O ou S;

$R^5$ est un alcoyle ($C_1$—$C_4$) ou un phényl-alcoyle ($C_1$—$C_4$), et

$R^6$ est un alcoyle ($C_1$—$C_4$) ou un phényl-alcoyle ($C_1$—$C_4$); et les dérivés convenant en agronomie des composés de formule I qui sont des sels de métal et d'ammonium, des sels d'addition d'acides et des sels d'addition d'ammonium quaternaire.

2. Un composé comme revendiqué dans la revendication 1 où $R^1$ est un phényle ou un phényle substitué par jusqu'à trois substituants choisis parmi un halogène, un alcoyle ($C_1$—$C_4$), un alcoxy ($C_1$—$C_4$), un trihalogénométhyle, un nitro et un cyano;

Y est $Z^1R^3$ ou $NR^7R^8$ où $Z^1$ est 0, $R^3$ est un alcoyle ($C_1$—$C_4$), un cation de métal alcalin, un cation de métal alcalino-terreux ou un ammonium ou un ammonium alcoyl-substitué; et $R^7$ et $R^8$ sont chacun un alcoyle ($C_1$—$C_4$) ou $R^7$ est un hydrogène et $R^8$ est $—CH_2C(O)OR^9$ où $R^9$ est un cation de métal alcalin ou un méthyle;

$R^5$ est un alcoyle ($C_1$—$C_4$); et

$R^6$ est un alcoyle ($C_1$—$C_4$).

3. Un composé comme revendiqué dans la revendication 1 où $R^1$ est un phényle éventuellement substitué par un substituant qui est Cl, Br, I, F, CN, $CF_3$, méthoxy ou éthoxy ou deux substituants qui sont tous deux Cl ou qui sont chacun un de Cl et $CF_3$.

4. Un composé comme revendiqué dans la revendication 1, où:

$R^1$ est un phényle, un 4-chlorophényle, un 4-bromophényle, un 4-fluorophényle, un 3,4-dichlorophényle, un 4-tri-fluorométhylphényle ou un 4-iodophényle;

Y est $Z^1R^3$ ou $Z^1$ est 0 et $R^3$ est un alcoyle ($C_1$—$C_4$) ou un cation de métal alcalin;

$R^5$ est un alcoyle ($C_2$—$C_4$); et

$R^6$ est un alcoyle ($C_2$—$C_4$).

5. Un composé comme revendiqué dans la revendication 1 dans lequel $R^1$ est un phényle, Y est ONa ou OK et $R^5$ et $R^6$ sont tous deux $C_2H_5$.

6. Un composé comme revendiqué dans la revendication 1 dans lequel $R^1$ est un 4-bromophényle ou un 3-bromophényle, Y est ONa ou OK et $R^5$ et $R^6$ sont tous deux $C_2H_5$.

7. Un composé comme revendiqué dans la revendication 1 où $R^1$ est un 4-chlorophényle, Y est ONa ou OK et $R^5$ et $R^6$ sont tous deux $C_3H_7$.

8. Un composé comme revendiqué dans la revendication 1 où $R^1$ est un 2-chlorophényle, un 4-chlorophényle ou un 3,4-dichlorophényle, Y est ONa, OK, $OCH_3$ ou $OCH_2CH_2OH$, et $R^5$ et $R^6$ sont tous deux $C_2H_5$.

9. Un composé comme revendiqué dans la revendication 1 où $R^1$ est un 4-fluorophényle, Y est OK ou ONa et $R^5$ et $R^6$ sont tous deux $C_2H_5$.

10. Un composé comme revendiqué dans la revendication 1 où $R^1$ est un 4-iodophényle, Y est OK ou ONa et $R^5$ et $R^6$ sont tous deux $C_2H_5$.

11. Un composé comme revendiqué dans la revendication 1 où $R^1$ est un 3-trifluorométhylphényle, un 4-trifluorométhylphényle ou 3,5-dichlorophényle, Y est OK ou ONa et $R^5$ et $R^6$ sont tous deux $C_2H_5$.

12. Un composé comme revendiqué dans la revendication 1 où $R^1$ est un 4-méthoxyphényle, Y est OK ou ONa et $R^5$ et $R^6$ sont tous deux $C_2H_5$.

13. Un composé comme revendiqué dans la revendication 1 où $R^1$ est un 2,4-dichlorophényle, Y est OK ou ONa et $R^5$ et $R^6$ sont tous deux $C_2H_5$.

14. Un composé comme revendiqué dans la revendication 1 où $R^1$ est un 4-chlorophényle, Y est OK ou ONa et $R^5$ et $R^6$ sont tous deux $CH_3$.

15. Un procédé de régulation de la croissance d'une plante graminée cultivée qui comprend l'application à la plante en croissance ou à ses semences d'une quantité régulatrice de croissance d'un ou plusieurs composés comme revendiqué dans l'une quelconque des revendications 1 à 14.

16. Un procédé comme revendiqué dans la revendication 15 appliqué à la production de semences hybrides par emploi d'au moins un composé régulateur de croissance qui induit une stérilité-mâle sans détruire la fertilité-femelle.

17. Un procédé comme revendiqué dans la revendication 16 dans lequel la plante est le maïs, l'orge, le blé, le sorgho ou une plante fourragère cultivée.

18. Un procédé comme revendiqué dans la revendication 16 ou 17 qui comprend le stade de traitement d'un parent mâle/femelle de la plante graminée, avant la méiose, avec le composé de régulation de la croissance.

19. Une composition de régulation de la croissance des plantes qui comprend un composé comme revendiqué dans l'une quelconque des revendications 1 à 14 et un support ou diluant convenant en agronomie de celui-ci.

**Revendications pour l'Etat contractant: AT**

1. Une composition de régulation de la croissance des plantes contenant comme ingrédient actif une pyridazine substituée ou un dérivé convenant en agronomie de celle-ci qui est un sel de métal ou d'ammonium, un sel d'addition d'acide ou un sel d'addition d'ammonium quaternaire, la composition contenant également un diluant ou support convenant en agronomie de l'ingredient actif, caractérisée en ce que la pyridazine substituée a pour formule:

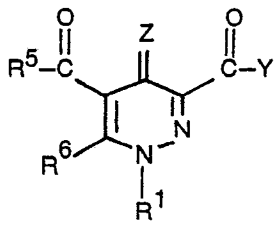

(I)

dans laquelle:

$R^1$ est un phényle ou un naphtyle substitués chacun éventuellement par jusqu'à trois substituants semblables ou différents choisis parmi un halogène, un alcoyle ($C_1$—$C_4$), un alcoxy ($C_1$—$C_4$), un éthoxycarbonyle, un trihalogénométhyle, un nitro et un cyano;

Y est $Z^1R^3$ ou $NR^7R^8$ où $Z^1$ est O ou S; $R^3$ est un hydrogène, un alcoyle ($C_1$—$C_6$), un 2-hydroxyéthyle, un alcoxy ($C_1$—$C_4$) alcoyle ($C_1$—$C_4$), un cycloalcoyle ($C_3$—$C_6$), un cycloalcoyl ($C_3$—$C_6$) alcoyle ($C_1$—$C_4$), un halogénoalcoyle ($C_1$—$C_3$) contenant jusqu'à 3 atomes d'halogène, un phényle ou un benzyle; et $R^7$ et $R^8$ sont (a) semblables ou différents et choisis parmi un hydrogène, un alcoyle ($C_1$—$C_4$), un alcoyle ($C_1$—$C_4$) substitué par un groupe carboxyle et un alcoxy ($C_1$—$C_4$) substitué par un groupe carboxyle ou (b) sont réunis et forment un groupe alcoylène ayant jusqu'à 5 atomes de carbone;

Z est O ou S;

$R^5$ est un alcoyle ($C_1$—$C_4$) ou un phényl-alcoyle ($C_1$—$C_4$), et

$R^6$ est un alcoyle ($C_1$—$C_4$) ou un phényl-alcoyle ($C_1$—$C_4$).

2. Une composition comme revendiqué dans la revendication 1 où $R^1$ est un phényle ou un phényle substitué par jusqu'à trois substituants choisis parmi un halogène, un alcoyle ($C_1$—$C_4$), un alcoxy ($C_1$—$C_4$), un trihalogénométhyle, un nitro et un cyano;

Y est $Z^1R^3$ ou $NR^7R^8$ où $Z^1$ est 0, $R^3$ est un alcoyle ($C_1$—$C_4$), un cation de métal alcalin, un cation de métal alcalino-terreux ou un ammonium ou un ammonium alcoyl-substitué; et $R^7$ et $R^8$ sont chacun un alcoyle ($C_1$—$C_4$) ou $R^7$ est un hydrogène et $R^8$ est —$CH_2C(O)OR^9$ où $R^9$ est un cation de métal alcalin ou un méthyle;

$R^5$ est un alcoyle ($C_1$—$C_4$); et

$R^6$ est un alcoyle ($C_1$—$C_4$).

3. Une composition comme revendiqué dans la revendication 1 où $R^1$ est un phényle éventuellement substitué par un substituant qui est Cl, Br, I, F, CN, $CF_3$, méthoxy ou éthoxy ou deux substituants qui sont tous deux Cl ou qui sont chacun un de Cl et $CF_3$.

4. Une composition comme revendiqué dans la revendication 1, où:

$R^1$ est un phényle, un 4-chlorophényle, un 4-bromophényle, un 4-fluorophényle, un 3,4-dichlorophényle, un 4-tri-fluorométhylphényle ou un 4-iodophényle;

Y est $Z^1R^3$ où $Z^1$ est 0 et $R^3$ est un alcoyle ($C_1$—$C_4$) ou un cation de métal alcalin;

$R^5$ est un alcoyle ($C_2$—$C_4$); et

$R^6$ est un alcoyle ($C_2$—$C_4$).

5. Une composition comme revendiqué dans la revendication 1 dans laquel $R^1$ est un phényle, Y est ONa ou OK et $R^5$ et $R^6$ sont tous deux $C_2H_5$.

6. Une composition comme revendiqué dans la revendication 1 dans laquelle $R^1$ est un 4-bromophényle ou un 3-bromophényle, Y est ONa ou OK et $R^5$ et $R^6$ sont tous deux $C_2H_5$.

7. Une composition comme revendiqué dans la revendication 1 où $R^1$ est un 4-chlorophényle, Y est ONa ou OK et $R^5$ et $R^6$ sont tous deux $C_3H_7$.

8. Une composition comme revendiqué dans la revendication 1 où $R^1$ est un 2-chlorophényle, un 4-chlorophényle ou un 3,4-dichlorophényle, Y est ONa, OK, $OCH_3$ ou $OCH_2CH_2OH$, et $R^5$ et $R^6$ sont tous deux $C_2H_5$.

9. Une composition comme revendiqué dans la revendication 1 où $R^1$ est un 4-fluorophényle, Y est OK ou ONa et $R^5$ et $R^6$ sont tous deux $C_2H_5$.

10. Une composition comme revendiqué dans la revendication 1 où $R^1$ est un 4-iodophényle, Y est OK ou ONa et $R^5$ et $R^6$ sont tous deux $C_2H_5$.

11. Une composition comme revendiqué dans la revendication 1 où $R^1$ est un 3-trifluorométhylphényle, un 4-trifluorométhylphényle ou un 3,5-dichlorophényle, Y est OK ou ONa et $R^5$ et $R^6$ sont tous deux $C_2H_5$.

12. Une composition comme revendiqué dans la revendication 1 où $R^1$ est un 4-méthoxyphényle, Y est OK ou ONa et $R^5$ et $R^6$ sont tous deux $C_2H_5$.

13. Une composition comme revendiqué dans la revendication 1 où $R^1$ est un 2,4-dichlorophényle, Y est OK ou ONa et $R^5$ et $R^6$ sont tous deux $CH_2H_5$.

14. Une composition comme reveneiqué dans la revendication 1 où $R^1$ est un 4-chlorophényle, Y est OK ou ONa et $R^5$ et $R^6$ sont tous deux $CH_3$.

15. Un procédé de régulation de la croissance d'une plante graminée cultivée qui comprend l'application à la plante en croissance ou à ses semences d'une quantité régulatrice de croissance d'une composition comme revendiqué dans l'une quelconque des revendications 1 à 14.

16. Un procédé comme revendiqué dans la revendication 15 appliqué à la production de semences

hybrides par emploi d'au moins un composé régulateur de croissance qui induit une stérilité-mâle sans détruire la fertilité-femelle.

17. Un procédé comme revendiqué dans la revendication 16 dans lequel la plante est le maïs, l'orge, le blé, le sorgho ou une plante fourragère cultivée.

18. Un procédé comme revendiqué dans la revendication 16 ou 17 qui comprend le stade de traitement d'un parent mâle/femelle de la plante graminée, avant la méiose, avec le composé de régulation de la croissance.